(19) Europäisches Patentamt
European Patent Office
Office européen des brevets

(11) Veröffentlichungsnummer: **0 599 135 A1**

(12) **EUROPÄISCHE PATENTANMELDUNG**

(21) Anmeldenummer: **93118206.7**

(51) Int. Cl.$^5$: **C07D 249/14**, A01N 43/653

(22) Anmeldetag: **10.11.93**

(30) Priorität: **23.11.92 DE 4239296**

(43) Veröffentlichungstag der Anmeldung: **01.06.94 Patentblatt 94/22**

(84) Benannte Vertragsstaaten: **BE CH DE ES FR GB IT LI NL**

(71) Anmelder: **BAYER AG**

**D-51368 Leverkusen(DE)**

(72) Erfinder: **Schallner, Otto, Dr.**
**Noldeweg 22**
**D-40789 Monheim(DE)**
Erfinder: **Haas, Wilhelm, Dr.**
**Schürgespfad 19**
**D-50259 Pulheim(DE)**
Erfinder: **Linker, Karl-Heinz**
**Albert-Schweitzer-Strasse 3**
**D-51377 Leverkusen(DE)**
Erfinder: **Findeisen, Kurt, Prof. Dr.**
**Dünfelder Strasse 26**
**D-51375 Leverkusen(DE)**
Erfinder: **König, Klaus, Dr.**
**Zum Hahnenberg 40**
**D-51519 Odenthal(DE)**
Erfinder: **Marhold, Albrecht, Dr.**
**Carl-Duisberg-Strasse 329**
**D-51373 Leverkusen(DE)**
Erfinder: **Santel, Hans-Joachim, Dr.**
**Grünstrasse 9a**
**D-51371 Leverkusen(DE)**
Erfinder: **Schmidt, Robert R.**
**Im Waldwinkel 110**
**D-51467 Bergisch Gladbach(DE)**

(54) **Substituierte Triazolinone.**

(57) Die Erfindung betrifft neue substituierte Triazolinone der allgemeinen Formel (I)

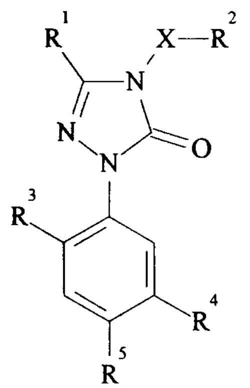

(I)

in welcher

| | |
|---|---|
| $R^1$ | für Wasserstoff, Alkyl, Alkoxy, Halogenalkyl oder Halogenalkoxy steht, |
| $R^2$ | fuhr Wasserstoff, Alkyl oder Halogenalkyl steht, |
| $R^3$ | für Wasserstoff oder Halogen steht, |
| $R^4$ | für Wasserstoff, Cyano, Halogen oder für einen Rest der Formel - O-$R^6$, -S-$R^6$, -C(O)-O-$R^6$, -C |

EP 0 599 135 A1

(O)-S-$R^6$, -$NR^6R^7$ oder -C(O)-$NR^6R^7$ steht,

- $R^5$ für Cyano oder Nitro steht und
- X für Sauerstoff oder Schwefel steht, wobei
- $R^6$ und $R^7$ unabhängig voneinander jeweils für Wasserstoff oder für jeweils gegebenenfalls substituiertes Alkyl, Alkenyl, Alkinyl, Cycloalkyl, Cycloalkylalkyl, Cycloalkoxycarbonyl, Aryl oder Arylalkyl stehen,

mehrere Verfahren zu ihrer Herstellung und ihre Verwendung als Herbizide.

Die Erfindung betrifft neue substituierte Triazolinone, mehrere Verfahren zu ihrer Herstellung und ihre Verwendung als Herbizide.

Es ist bekannt, daß bestimmte substituierte Triazolinone wie beispielsweise die Verbindung 3-Methyl-4-propargyl-1-(2,5-difluor-4-cyano-phenyl)-1,2,4-triazolin-5-on herbizide Eigenschaften besitzen (vergl. z.B. DE 38 39 480).

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriederstellend.

Es wurden neue substituierte Triazolinone der allgemeinen Formel (I),

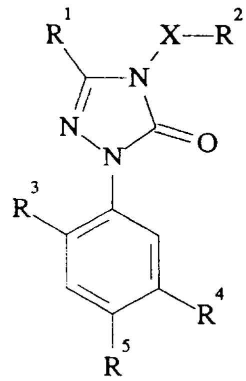

(I)

in welcher

| | |
|---|---|
| $R^1$ | für Wasserstoff, Alkyl, Alkoxy, Halogenalkyl oder Halogenalkoxy steht, |
| $R^2$ | für Wasserstoff, Alkyl oder Halogenalkyl steht, |
| $R^3$ | für Wasserstoff oder Halogen steht, |
| $R^4$ | für Wasserstoff, Cyano, Halogen oder für einen Rest der Formel - O-$R^6$, -S-$R^6$, -C(O)-O-$R^6$, -C(O)-S-$R^6$, -N$R^6R^7$ oder -C(O)-N$R^6R^7$ steht, |
| $R^5$ | für Cyano oder Nitro steht und |
| X | für Sauerstoff oder Schwefel steht, wobei |
| $R^6$ und $R^7$ | unabhängig voneinander jeweils für Wasserstoff oder für jeweils gegebenenfalls substituiertes Alkyl, Alkenyl, Alkinyl, Cycloalkyl, Cycloalkylalkyl, Cycloalkoxycarbonyl, Aryl oder Arylalkyl stehen, |

gefunden.

Die Verbindungen der Formel (I) können gegebenenfalls in Abhängigkeit von der Art der Substituenten als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazolinone der allgemeinen Formel (I),

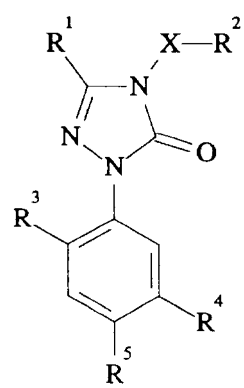

(I)

in welcher

$R^1$ für Wasserstoff, Alkyl, Alkoxy, Halogenalkyl oder Halogenalkoxy steht,

$R^2$ für Wasserstoff, Alkyl oder Halogenalkyl steht,

$R^3$ für Wasserstoff oder Halogen steht,

$R^4$ für Wasserstoff, Cyano, Halogen oder für einen Rest der Formel - O-$R^6$, -S-$R^6$, -C(O)-O-$R^6$, -C(O)-S-$R^6$, -$NR^6R^7$ oder -C(O)-$NR^6R^7$ steht,

$R^5$ für Cyano oder Nitro steht und

X für Sauerstoff oder Schwefel steht, wobei

$R^6$ und $R^7$ unabhängig voneinander jeweils für Wasserstoff oder für jeweils gegebenenfalls substituiertes Alkyl, Alkenyl, Alkinyl, Cycloalkyl, Cycloalkylalkyl, Cycloalkoxycarbonyl, Aryl oder Arylalkyl stehen,

erhält, wenn man

a) 1H-Triazolinone der Formel (II),

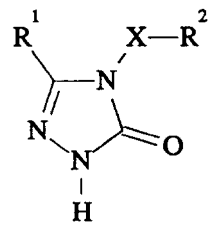

(II)

in welcher

$R^1$, $R^2$ und X die oben angegebenen Bedeutungen haben,

mit Halogenbenzol-Derivaten der Formel (III),

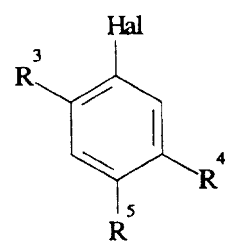

(III)

in welcher

$R^3$, $R^4$ und $R^5$ die oben angegebenen Bedeutungen haben und

Hal für Halogen steht,

gegebenenfalls in Gegenwart eines Verdünnungsmittels und gegebebenfalls in Gegenwart eines Reaktionshilfsmittels umsetzt, oder wenn man

b) substituierte Triazolinone der Formel (Ia),

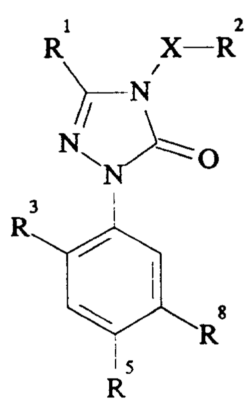

(Ia)

in welcher

$R^1$, $R^2$, $R^3$, $R^5$ und X die oben angegebenen Bedeutungen haben und

$R^8$ für Halogen steht,

mit Nukleophilen der Formel (IV),

$R^9$-Z-H (IV)

in welcher

Z für Sauerstoff, Schwefel oder für den Rest -N($R^7$)- steht,

$R^9$ für jeweils gegebenenfalls substituiertes Alkyl, Alkenyl, Alkinyl, Cycloalkyl oder Aryl steht und

$R^7$ die oben angegebene Bedeutung hat,

gegebenenfalls in Gegenwart eines Verdünnungsmittels und gegebenenfalls in Gegenwart eines Reaktionshilfsmittels umsetzt, oder wenn man

c) substituierte Triazolinone der Formel (V),

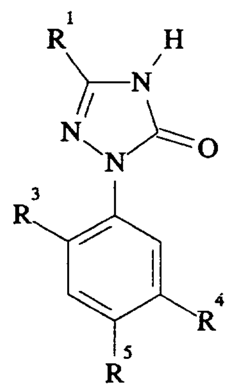

(V)

in welcher

$R^1$, $R^3$, $R^4$ und $R^5$ die oben angegebenen Bedeutungen haben,

mit Sulfenylierungsmitteln der Formel (VI),

$R^2$-S-Hal (VI)

in welcher

$R^2$ die oben angegebene Bedeutung hat und

Hal für Halogen steht,

gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolinone der allgemeinen Formel (I) herbizide Eigenschaften besitzen.

Überraschenderweise zeigen die erfindungsgemäßen substituierten Triazolinone der allgemeinen Formel (I) eine erheblich bessere herbizide Wirksamkeit gegenüber Problemunkräutern bei vergleichbar guter Nutzpflanzenselektivität im Vergleich zu den aus dem Stand der Technik bekannten substituierten Triazolinonen, wie beispielsweise die Verbindung 3-Methyl-4-propargyl-1-(2,5-difluor-4-cyano-phenyl)-1,2,4-triazolin-5-on, welche chemisch und wirkungsmäßig naheliegende Verbindungen sind.

Die erfindungsgemäßen substituierten Triazolinone sind durch die Formel (I) allgemein definiert. Bevorzugt sind Verbindungen der Formel (I), bei welchen

$R^1$ für Wasserstoff oder für jeweils geradkettiges oder verzweigtes Alkyl, Alkoxy, Halogenalkyl oder Halogenalkoxy mit jeweils 1 bis 8 Kohlenstoffatomen und gegebenenfalls 1 bis 17 gleichen oder verschiedenen Halogenatomen - insbesondere Fluor, Chlor, Brom oder Iod - steht,

$R^2$ für Wasserstoff oder für jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen - insbesondere Fluor, Chlor, Brom oder Iod - steht,

$R^3$ für Wasserstoff, Fluor, Chlor, Brom oder Iod steht,

$R^4$ für Wasserstoff, Cyano, Fluor, Chlor, Brom, Iod oder für einen Rest der Formel -O-$R^6$, -S-

$R^6$, -C(O)-O-$R^6$, -C(O)-S-$R^6$, -N$R^6R^7$ oder -C(O)-N$R^6R^7$ steht,

$R^5$ für Cyano oder Nitro steht und

X für Sauerstoff oder Schwefel steht, wobei

$R^6$ und $R^7$ unabhängig voneinander jeweils für Wasserstoff oder für gegebenenfalls einfach oder mehrfach, gleich oder verschieden substituiertes, geradkettiges oder verzweigtes Alkyl mit 1 bis 8 Kohlenstoffatomen stehen, wobei als Substituenten infrage kommen:
Halogen - insbesondere Fluor, Chlor, Brom und/oder Iod - , Cyano, Carboxyl, Carbamoyl, jeweils geradkettiges oder verzweigtes Alkoxy, Alkoxyalkoxy, Halogenalkoxy (wobei Halogen für Fluor, Chlor, Brom und/oder Iod steht), Alkylthio, Alkylsulfinyl, Alkylsulfonyl, Alkylcarbonyl, Alkoxycarbonyl, Alkenyloxycarbonyl, Alkinyloxycarbonyl, Alkoxyalkoxycarbonyl, N-Alkylaminocarbonyl, N,N-Dialkylaminocarbonyl, Trialkysilyl oder Alkylsulfonylaminocarbonyl mit jeweils bis zu 8 Kohlenstoffatomen in den einzelnen Alkyl-bzw. Alkenyl- oder Alkinylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht;

$R^6$ und $R^7$ außerdem für jeweils gegebenenfalls einfach oder mehrfach, gleich oder verschieden durch Fluor, Chlor, Brom und/oder Iod substituiertes Alkenyl oder Alkinyl mit jeweils 2 bis 8 Kohlenstoffatomen stehen;

$R^6$ und $R^7$ außerdem für jeweils gegebenenfalls im Cycloalkylteil einfach oder mehrfach, gleich oder verschieden durch Fluor, Chlor, Brom und/oder Iod und/oder jeweils geradkettiges oder verzweigtes Alkyl und/oder Alkoxy und/oder Alkoxycarbonyl mit jeweils 1 bis 4 Kohlenstoffatomen substituiertes Cycloalkyl, Cycloalkylalkyl oder Cycloalkyloxycarbonyl mit jeweils 3 bis 8 Kohlenstoffatomen im Cycloalkylteil und gegebenenfalls 1 bis 4 Kohlenstoffatomen im geradkettigen oder verzweigten Alkylteil stehen, oder

$R^6$ und $R^7$ für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil stehen, wobei als Arylsubstituenten jeweils infrage kommen:
Halogen, Cyano, Nitro, Hydroxy, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, geradkettiges oder verzweigtes Alkoxycarbonylalkyloxy mit 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl, wobei Halogen jeweils Fluor, Chlor, Brom und/oder Iod bedeutet.

Besonders bevorzugt sind Verbindungen der Formel (I), bei welchen

$R^1$ für Wasserstoff oder für jeweils geradkettiges oder verzweigtes Alkyl, Alkoxy, Halogenalkyl oder Halogenalkoxy mit jeweils 1 bis 6 Kohlenstoffatomen und gegebenenfalls 1 bis 13 gleichen oder verschiedenen Halogenatomen - insbesondere Fluor, Chlor oder Brom - steht,

$R^2$ für Wasserstoff oder für jeweils geradkettiges oder verzweigtes Alkyl oder Halogenalkyl mit jeweils 1 bis 6 Kohlenstoffatomen und gegebenenfalls 1 bis 13 gleichen oder verschiedenen Halogenatomen - insbesondere Fluor, Chlor oder Brom - steht,

$R^3$ für Wasserstoff, Fluor, Chlor oder Brom steht,

$R^4$ für Wasserstoff, Cyano, Fluor, Chlor, Brom oder für einen Rest der Formel -O-$R^6$, -S-$R^6$, -C(O)-O-$R^6$, -C(O)-S-$R^6$, -N$R^6R^7$ oder -C(O)-N$R^6R^7$ steht,

$R^5$ für Cyano oder Nitro steht und

X für Sauerstoff oder Schwefel steht, wobei

$R^6$ und $R^7$ unabhängig voneinander jeweils für Wasserstoff oder für gegebenenfalls einfach bis zweifach, gleich oder verschieden substituiertes, geradkettiges oder verzweigtes Alkyl mit 1 bis 6 Kohlenstoffatomen stehen, wobei als Substituenten infrage kommen:
Cyano, Carboxyl, Carbamoyl, jeweils geradkettiges oder verzweigtes Alkoxy, Alkoxyalkoxy, Halogenalkoxy (wobei Halogen für Fluor, Chlor und/oder Brom steht), Alkylthio,

Alkylsulfinyl, Alkylsulfonyl, Alkylcarbonyl, Alkoxycarbonyl, Alkenyloxycarbonyl, Alkinyloxycarbonyl, Alkoxyalkoxycarbonyl, N-Alkylaminocarbonyl, N,N-Dialkylaminocarbonyl, Trialkylsilyl oder Alkylsulfonylaminocarbonyl mit jeweils bis zu 6 Kohlenstoffatomen in den einzelnen Alkyl- bzw. Alkenyl- oder Alkinylteilen oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff, Sauerstoff und/oder Schwefel - steht;

$R^6$ und $R^7$ außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - stehen;

$R^6$ und $R^7$ außerdem für jeweils gegebenenfalls einfach bis fünffach, gleich oder verschieden durch Fluor, Chlor und/oder Brom substituiertes Alkenyl oder Alkinyl mit jeweils 2 bis 6 Kohlenstoffatomen stehen;

$R^6$ und $R^7$ außerdem für jeweils gegebenenfalls im Cycloalkylteil einfach bis vierfach, gleich oder verschieden durch Fluor, Chlor und/oder Brom und/oder jeweils geradkettiges oder verzweigtes Alkyl und/oder Alkoxy und/oder Alkoxycarbonyl mit jeweils 1 bis 3 Kohlenstoffatomen substituiertes Cycloalkyl, Cycloalkylalkyl oder Cycloalkyloxycarbonyl mit jeweils 3 bis 7 Kohlenstoffatomen im Cycloalkylteil und gegebenenfalls 1 bis 3 Kohlenstoffatomen im geradkettigen oder verzweigten Alkylteil stehen, oder

$R^6$ und $R^7$ für jeweils gegebenenfalls im Arylteil einfach bis fünffach, gleich oder verschieden substituiertes Arylalkyl oder Aryl mit jeweils 6 oder 10 Kohlenstoffatomen im Arylteil und gegebenenfalls 1 bis 3 Kohlenstoffatomen im geradkettigen oder verzweigten Alkylteil stehen, wobei als Arylsubstituenten jeweils infrage kommen:

Halogen, Cyano, Nitro, Hydroxy, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, geradkettiges oder verzweigtes Alkoxycarbonylalkyloxy mit 1 bis 4 Kohlenstoffatomen in den einzelnen Alkylteilen sowie gegebenenfalls einfach bis fünffach, gleich oder verschieden durch Halogen und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl, wobei Halogen jeweils Fluor, Chlor und/oder Brom bedeutet.

Ganz besonders bevorzugt sind Verbindungen der Formel (I), bei welchen

$R^1$ für Wasserstoff oder für jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen - insbesondere Fluor, Chlor oder Brom - steht,

$R^2$ für Wasserstoff oder für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen - insbesondere Fluor, Chlor oder Brom - steht,

$R^3$ für Wasserstoff, Fluor, Chlor oder Brom steht,

$R^4$ für Wasserstoff, Cyano, Fluor, Chlor, Brom oder für einen Rest der Formel $-O-R^6$, $-S-R^6$, $-C(O)-O-R^6$, $-C(O)-S-R^6$, $-NR^6R^7$ oder $-C(O)-NR^6R^7$ steht,

$R^5$ für Cyano oder Nitro steht und

X für Sauerstoff oder Schwefel steht, wobei

$R^6$ und $R^7$ unabhängig voneinander jeweils für Wasserstoff oder für gegebenenfalls einfach substituiertes, geradkettiges oder verzweigtes Alkyl mit 1 bis 4 Kohlenstoffatomen stehen, wobei als Substituenten infrage kommen:

Cyano, Carboxyl, Carbamoyl, jeweils geradkettiges oder verzweigtes Alkoxy, Alkoxyalkoxy, Halogenalkoxy (wobei Halogen für Fluor, Chlor und/oder Brom steht), Alkylthio, Alkylsulfinyl, Alkylsulfonyl, Alkylcarbonyl, Alkoxycarbonyl, Alkenyloxycarbonyl, Alkinyloxycarbonyl, Alkoxyalkoxycarbonyl, N-Alkylaminocarbonyl, N,N-Dialkylaminocarbonyl, Trialkylsilyl oder Alkylsulfonylaminocarbonyl mit jeweils bis zu 4 Kohlenstoffatomen in den einzelnen Alkyl- bzw. Alkenyl- oder Alkinylteilen, oder Heterocyclyl, wobei als Heterocyclylrest ein fünf- oder sechsgliedriger, gesättigter oder ungesättigter Heterocyclus mit 1 bis 3 gleichen oder verschiedenen Heteroatomen - insbesondere Stickstoff, Sauerstoff

und/oder Schwefel - steht;

$R^6$ und $R^7$ außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - stehen;

$R^6$ und $R^7$ außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 3 bis 5 Kohlenstoffatomen stehen;

$R^6$ und $R^7$ außerdem für jeweils gegebenenfalls im Cycloalkylteil einfach oder zweifach, gleich oder verschieden durch Fluor, Chlor, Brom, Methyl, Ethyl, Methoxy, Ethoxy, Methoxycarbonyl und/oder Ethoxycarbonyl substituiertes Cycloalkyl, Cycloalkylalkyl oder Cycloalkyloxycarbonyl mit jeweils 3 bis 6 Kohlenstoffatomen im Cycloalkylteil und gegebenenfalls 1 oder 2 Kohlenstoffatomen im Alkylteil stehen, oder

$R^6$ und $R^7$ für jeweils gegebenenfalls im Phenylteil einfach bis dreifach, gleich oder verschieden substituiertes Phenylalkyl oder Phenyl mit gegebenenfalls 1 oder 2 Kohlenstoffatomen im Alkylteil stehen, wobei als Phenylsubstituenten jeweils infrage kommen:
Halogen, Cyano, Nitro, Hydroxy, jeweils geradkettiges oder verzweigtes Alkyl, Alkoxy, Alkylthio, Alkylsulfinyl oder Alkylsulfonyl mit jeweils 1 bis 3 Kohlenstoffatomen oder jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen sowie geradkettiges oder verzweigtes Alkoxycarbonylalkyloxy mit 1 bis 2 Kohlenstoffatomen in den einzelnen Alkylteilen, wobei Halogen jeweils Fluor, Chlor und/oder Brom bedeutet.

Im einzelnen seien außer den bei den Herstellungsbeispielen aufgeführten Verbindungen die folgenden substituierten Triazolinone der allgemeinen Formel (I) genannt:

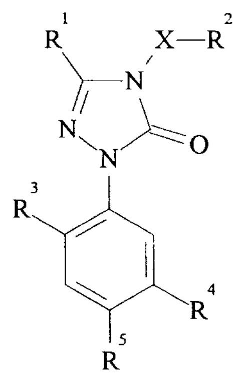

(I)

$R^5$ = CN und $NO_2$

| $R^1$ | $R^2$ | $R^3$ | $R^4$ | X |
|---|---|---|---|---|
| $CH_3$ | H | F | $-O-CH_2-C{\equiv}CH$ | O |
| $CH_3$ | H | F | $-O-C_2H_5$ | O |
| $CH_3$ | H | F | $-O-CH_3$ | O |
| $CH_3$ | H | F | $-S-CH_3$ | O |
| $CH_3$ | H | F | $-O-CH_2-CF_3$ | O |
| $CH_3$ | H | F | $-O-CH(CH_2F)_2$ | O |
| $CH_3$ | H | F | $-O-(CH_2-CH_2-O)_2-CH_3$ | O |

| $R^1$ | $R^2$ | $R^3$ | $R^4$ | X |
|---|---|---|---|---|
| $CH_3$ | H | F | $-O-CH_2-CH_2-O-C_2H_5$ | O |
| $CH_3$ | H | F | $-O-CH(CH_3)-COOC_2H_5$ | O |
| $CH_3$ | H | F | $-O-CH_2-COO-n-C_4H_9$ | O |
| $CH_3$ | H | F | $-O-CH_2-CH=CH_2$ | O |
| $CH_3$ | H | F | $-O-CH(CH_3)-C\equiv CH$ | O |
| $CH_3$ | H | F | $-S-CH_2-COOCH_3$ | O |
| $CH_3$ | H | F | $-S-CH_2-C\equiv CH$ | O |
| $CH_3$ | H | F | $-S-C_2H_5$ | O |
| $CH_3$ | H | F | $-O-i-C_3H_7$ | O |
| $CH_3$ | H | F | $-CH_2-CN$ | O |
| $CH_3$ | H | F | $-O-CH(CH_3)-COO-CH_2-C\equiv CH$ | O |
| $CH_3$ | H | F | 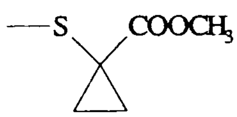 | O |
| $CH_3$ | H | F | 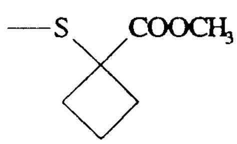 | O |
| $CF_3$ | $CH_3$ | H | $-O-CH_2-C\equiv CH$ | O |
| $CF_3$ | $CH_3$ | H | $-O-C_2H_5$ | O |

| $R^1$ | $R^2$ | $R^3$ | $R^4$ | X |
|---|---|---|---|---|
| $CF_3$ | $CH_3$ | H | $-O-CH_3$ | O |
| $CF_3$ | $CH_3$ | H | $-S-CH_3$ | O |
| $CF_3$ | $CH_3$ | H | $-O-CH_2-CF_3$ | O |
| $CF_3$ | $CH_3$ | H | $-O-CH(CH_2F)_2$ | O |
| $CF_3$ | $CH_3$ | H | $-O-(CH_2-CH_2-O)_2-CH_3$ | O |
| $CF_3$ | $CH_3$ | H | $-O-CH_2-CH_2-O-C_2H_5$ | O |
| $CF_3$ | $CH_3$ | H | $-O-CH(CH_3)-COOC_2H_5$ | O |
| $CF_3$ | $CH_3$ | H | $-O-CH_2-COO-n-C_4H_9$ | O |
| $CF_3$ | $CH_3$ | H | $-O-CH_2-CH=CH_2$ | O |
| $CF_3$ | $CH_3$ | H | $-O-CH(CH_3)-C\equiv CH$ | O |
| $CF_3$ | $CH_3$ | H | $-S-CH_2-COOCH_3$ | O |
| $CF_3$ | $CH_3$ | H | $-S-CH_2-C\equiv CH$ | O |
| $CF_3$ | $CH_3$ | H | $-S-C_2H_5$ | O |
| $CF_3$ | $CH_3$ | H | $-O-i-C_3H_7$ | O |
| $CF_3$ | $CH_3$ | H | $-CH_2-CN$ | O |
| $CF_3$ | $CH_3$ | H | $-O-CH(CH_3)-COO-CH_2-C\equiv CH$ | O |
| $CF_3$ | $CH_3$ | H | 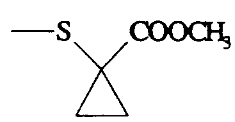 | O |

| $R^1$ | $R^2$ | $R^3$ | $R^4$ | X |
|---|---|---|---|---|
| $CF_3$ | $CH_3$ | H | 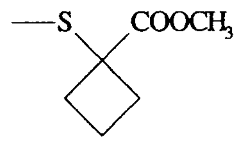 | O |
| $CF_3$ | $CH_3$ | Cl | $-O-CH_2-C{\equiv}CH$ | O |
| $CF_3$ | $CH_3$ | Cl | $-O-C_2H_5$ | O |
| $CF_3$ | $CH_3$ | Cl | $-O-CH_3$ | O |
| $CF_3$ | $CH_3$ | Cl | $-S-CH_3$ | O |
| $CF_3$ | $CH_3$ | Cl | $-O-CH_2-CF_3$ | O |
| $CF_3$ | $CH_3$ | Cl | $-O-CH(CH_2F)_2$ | O |
| $CF_3$ | $CH_3$ | Cl | $-O-(CH_2-CH_2-O)_2-CH_3$ | O |
| $CF_3$ | $CH_3$ | Cl | $-O-CH_2-CH_2-O-C_2H_5$ | O |
| $CF_3$ | $CH_3$ | Cl | $-O-CH(CH_3)-COOC_2H_5$ | O |
| $CF_3$ | $CH_3$ | Cl | $-O-CH_2-COO-n-C_4H_9$ | O |
| $CF_3$ | $CH_3$ | Cl | $-O-CH_2-CH{=}CH_2$ | O |
| $CF_3$ | $CH_3$ | Cl | $-O-CH(CH_3)-C{\equiv}CH$ | O |
| $CF_3$ | $CH_3$ | Cl | $-S-CH_2-COOCH_3$ | O |
| $CF_3$ | $CH_3$ | Cl | $-S-CH_2-C{\equiv}CH$ | O |
| $CF_3$ | $CH_3$ | Cl | $-S-C_2H_5$ | O |

| $R^1$ | $R^2$ | $R^3$ | $R^4$ | X |
|---|---|---|---|---|
| $CF_3$ | $CH_3$ | Cl | $-O-i-C_3H_7$ | O |
| $CF_3$ | $CH_3$ | Cl | $-CH_2-CN$ | O |
| $CF_3$ | $CH_3$ | Cl | $-O-CH(CH_3)-COO-CH_2-C\equiv CH$ | O |
| $CF_3$ | $CH_3$ | Cl | 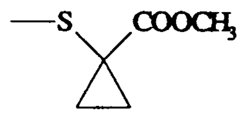 | O |
| $CF_3$ | $CH_3$ | Cl | 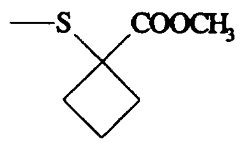 | O |
| $CF_3$ | $CH_3$ | F | $-O-CH_2-C\equiv CH$ | O |
| $CF_3$ | $CH_3$ | F | $-O-C_2H_5$ | O |
| $CF_3$ | $CH_3$ | F | $-O-CH_3$ | O |
| $CF_3$ | $CH_3$ | F | $-S-CH_3$ | O |
| $CF_3$ | $CH_3$ | F | $-O-CH_2-CF_3$ | O |
| $CF_3$ | $CH_3$ | F | $-O-CH(CH_2F)_2$ | O |
| $CF_3$ | $CH_3$ | F | $-O-(CH_2-CH_2-O)_2-CH_3$ | O |
| $CF_3$ | $CH_3$ | F | $-O-CH_2-CH_2-O-C_2H_5$ | O |
| $CF_3$ | $CH_3$ | F | $-O-CH(CH_3)-COOC_2H_5$ | O |
| $CF_3$ | $CH_3$ | F | $-O-CH_2-COO-n-C_4H_9$ | O |

| $R^1$ | $R^2$ | $R^3$ | $R^4$ | X |
|---|---|---|---|---|
| $CF_3$ | $CH_3$ | F | $-O-CH_2-CH=CH_2$ | O |
| $CF_3$ | $CH_3$ | F | $-O-CH(CH_3)-C\equiv CH$ | O |
| $CF_3$ | $CH_3$ | F | $-S-CH_2-COOCH_3$ | O |
| $CF_3$ | $CH_3$ | F | $-S-CH_2-C\equiv CH$ | O |
| $CF_3$ | $CH_3$ | F | $-S-C_2H_5$ | O |
| $CF_3$ | $CH_3$ | F | $-O-i-C_3H_7$ | O |
| $CF_3$ | $CH_3$ | F | $-CH_2-CN$ | O |
| $CF_3$ | $CH_3$ | F | $-O-CH(CH_3)-COO-CH_2-C\equiv CH$ | O |
| $CF_3$ | $CH_3$ | F | 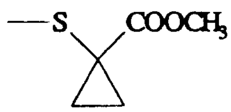 | O |
| $CF_3$ | $CH_3$ | F | 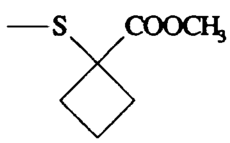 | O |
| $CH_3$ | $CHF_2$ | Cl | $-O-CH_2-C\equiv CH$ | O |
| $CH_3$ | $CHF_2$ | Cl | $-O-C_2H_5$ | O |
| $CH_3$ | $CHF_2$ | Cl | $-O-CH_3$ | O |
| $CH_3$ | $CHF_2$ | Cl | $-S-CH_3$ | O |
| $CH_3$ | $CHF_2$ | Cl | $-O-CH_2-CF_3$ | O |

| $R^1$ | $R^2$ | $R^3$ | $R^4$ | X |
|---|---|---|---|---|
| $CH_3$ | $CHF_2$ | Cl | $-O-CH(CH_2F)_2$ | O |
| $CH_3$ | $CHF_2$ | Cl | $-O-(CH_2-CH_2-O)_2-CH_3$ | O |
| $CH_3$ | $CHF_2$ | Cl | $-O-CH_2-CH_2-O-C_2H_5$ | O |
| $CH_3$ | $CHF_2$ | Cl | $-O-CH(CH_3)-COOC_2H_5$ | O |
| $CH_3$ | $CHF_2$ | Cl | $-O-CH_2-COO-n-C_4H_9$ | O |
| $CH_3$ | $CHF_2$ | Cl | $-O-CH_2-CH=CH_2$ | O |
| $CH_3$ | $CHF_2$ | Cl | $-O-CH(CH_3)-C\equiv CH$ | O |
| $CH_3$ | $CHF_2$ | Cl | $-S-CH_2-COOCH_3$ | O |
| $CH_3$ | $CHF_2$ | Cl | $-S-CH_2-C\equiv CH$ | O |
| $CH_3$ | $CHF_2$ | Cl | $-S-C_2H_5$ | O |
| $CH_3$ | $CHF_2$ | Cl | $-O-i-C_3H_7$ | O |
| $CH_3$ | $CHF_2$ | Cl | $-CH_2-CN$ | O |
| $CH_3$ | $CHF_2$ | Cl | $-O-CH(CH_3)-COO-CH_2-C\equiv CH$ | O |
| $CH_3$ | $CHF_2$ | Cl | 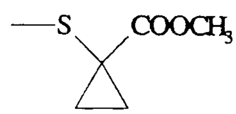 | O |
| $CH_3$ | $CHF_2$ | Cl | 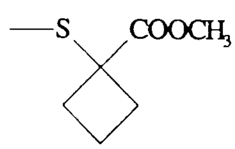 | O |

| $R^1$ | $R^2$ | $R^3$ | $R^4$ | X |
|---|---|---|---|---|
| $CH_3$ | $CHF_2$ | F | $-O-CH_2-C\equiv CH$ | O |
| $CH_3$ | $CHF_2$ | F | $-O-C_2H_5$ | O |
| $CH_3$ | $CHF_2$ | F | $-O-CH_3$ | O |
| $CH_3$ | $CHF_2$ | F | $-S-CH_3$ | O |
| $CH_3$ | $CHF_2$ | F | $-O-CH_2-CF_3$ | O |
| $CH_3$ | $CHF_2$ | F | $-O-CH(CH_2F)_2$ | O |
| $CH_3$ | $CHF_2$ | F | $-O-(CH_2-CH_2-O)_2-CH_3$ | O |
| $CH_3$ | $CHF_2$ | F | $-O-CH_2-CH_2-O-C_2H_5$ | O |
| $CH_3$ | $CHF_2$ | F | $-O-CH(CH_3)-COOC_2H_5$ | O |
| $CH_3$ | $CHF_2$ | F | $-O-CH_2-COO-n-C_4H_9$ | O |
| $CH_3$ | $CHF_2$ | F | $-O-CH_2-CH=CH_2$ | O |
| $CH_3$ | $CHF_2$ | F | $-O-CH(CH_3)-C\equiv CH$ | O |
| $CH_3$ | $CHF_2$ | F | $-S-CH_2-COOCH_3$ | O |
| $CH_3$ | $CHF_2$ | F | $-S-CH_2-C\equiv CH$ | O |
| $CH_3$ | $CHF_2$ | F | $-S-C_2H_5$ | O |
| $CH_3$ | $CHF_2$ | F | $-O-i-C_3H_7$ | O |
| $CH_3$ | $CHF_2$ | F | $-CH_2-CN$ | O |
| $CH_3$ | $CHF_2$ | F | $-O-CH(CH_3)-COO-CH_2-C\equiv CH$ | O |

| $R^1$ | $R^2$ | $R^3$ | $R^4$ | X |
|---|---|---|---|---|
| $CH_3$ | $CHF_2$ | F | 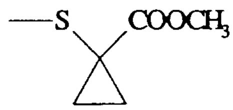 | O |
| $CH_3$ | $CHF_2$ | F | 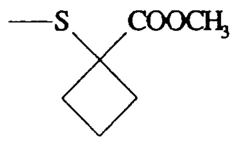 | O |
| $CF_3$ | $CHF_2$ | F | $-O-CH_2-C\equiv CH$ | O |
| $CF_3$ | $CHF_2$ | F | $-O-C_2H_5$ | O |
| $CF_3$ | $CHF_2$ | F | $-O-CH_3$ | O |
| $CF_3$ | $CHF_2$ | F | $-S-CH_3$ | O |
| $CF_3$ | $CHF_2$ | F | $-O-CH_2-CF_3$ | O |
| $CF_3$ | $CHF_2$ | F | $-O-CH(CH_2F)_2$ | O |
| $CF_3$ | $CHF_2$ | F | $-O-(CH_2-CH_2-O)_2-CH_3$ | O |
| $CF_3$ | $CHF_2$ | F | $-O-CH_2-CH_2-O-C_2H_5$ | O |
| $CF_3$ | $CHF_2$ | F | $-O-CH(CH_3)-COOC_2H_5$ | O |
| $CF_3$ | $CHF_2$ | F | $-O-CH_2-COO-n-C_4H_9$ | O |
| $CF_3$ | $CHF_2$ | F | $-O-CH_2-CH=CH_2$ | O |
| $CF_3$ | $CHF_2$ | F | $-O-CH(CH_3)-C\equiv CH$ | O |
| $CF_3$ | $CHF_2$ | F | $-S-CH_2-COOCH_3$ | O |

| $R^1$ | $R^2$ | $R^3$ | $R^4$ | X |
|---|---|---|---|---|
| $CF_3$ | $CHF_2$ | F | $-S-CH_2-C\equiv CH$ | O |
| $CF_3$ | $CHF_2$ | F | $-S-C_2H_5$ | O |
| $CF_3$ | $CHF_2$ | F | $-O-i-C_3H_7$ | O |
| $CF_3$ | $CHF_2$ | F | $-CH_2-CN$ | O |
| $CF_3$ | $CHF_2$ | F | $-O-CH(CH_3)-COO-CH_2-C\equiv CH$ | O |
| $CF_3$ | $CHF_2$ | F | 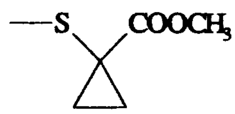 | O |
| $CF_3$ | $CHF_2$ | F | 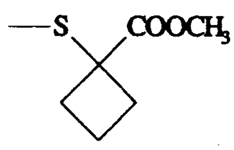 | O |
| H | $CHF_2$ | F | $-O-CH_2-C\equiv CH$ | O |
| H | $CHF_2$ | F | $-O-C_2H_5$ | O |
| H | $CHF_2$ | F | $-O-CH_3$ | O |
| H | $CHF_2$ | F | $-S-CH_3$ | O |
| H | $CHF_2$ | F | $-O-CH_2-CF_3$ | O |
| H | $CHF_2$ | F | $-O-CH(CH_2F)_2$ | O |
| H | $CHF_2$ | F | $-O-(CH_2-CH_2-O)_2-CH_3$ | O |
| H | $CHF_2$ | F | $-O-CH_2-CH_2-O-C_2H_5$ | O |

| $R^1$ | $R^2$ | $R^3$ | $R^4$ | X |
|---|---|---|---|---|
| H | $CHF_2$ | F | $-O-CH(CH_3)-COOC_2H_5$ | O |
| H | $CHF_2$ | F | $-O-CH_2-COO-n-C_4H_9$ | O |
| H | $CHF_2$ | F | $-O-CH_2-CH=CH_2$ | O |
| H | $CHF_2$ | F | $-O-CH(CH_3)-C\equiv CH$ | O |
| H | $CHF_2$ | F | $-S-CH_2-COOCH_3$ | O |
| H | $CHF_2$ | F | $-S-CH_2-C\equiv CH$ | O |
| H | $CHF_2$ | F | $-S-C_2H_5$ | O |
| H | $CHF_2$ | F | $-O-i-C_3H_7$ | O |
| H | $CHF_2$ | F | $-CH_2-CN$ | O |
| H | $CHF_2$ | F | $-O-CH(CH_3)-COO-CH_2-C\equiv CH$ | O |
| H | $CHF_2$ | F | 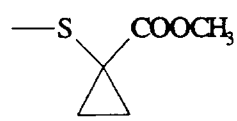 | O |
| H | $CHF_2$ | F | 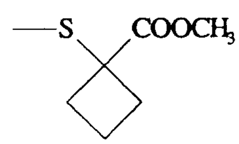 | O |
| H | $CH_3$ | F | $-O-CH_2-C\equiv CH$ | O |
| H | $CH_3$ | F | $-O-C_2H_5$ | O |
| H | $CH_3$ | F | $-O-CH_3$ | O |

| $R^1$ | $R^2$ | $R^3$ | $R^4$ | X |
|---|---|---|---|---|
| H | $CH_3$ | F | $-S-CH_3$ | O |
| H | $CH_3$ | F | $-O-CH_2-CF_3$ | O |
| H | $CH_3$ | F | $-O-CH(CH_2F)_2$ | O |
| H | $CH_3$ | F | $-O-(CH_2-CH_2-O)_2-CH_3$ | O |
| H | $CH_3$ | F | $-O-CH_2-CH_2-O-C_2H_5$ | O |
| H | $CH_3$ | F | $-O-CH(CH_3)-COOC_2H_5$ | O |
| H | $CH_3$ | F | $-O-CH_2-COO-n-C_4H_9$ | O |
| H | $CH_3$ | F | $-O-CH_2-CH=CH_2$ | O |
| H | $CH_3$ | F | $-O-CH(CH_3)-C\equiv CH$ | O |
| H | $CH_3$ | F | $-S-CH_2-COOCH_3$ | O |
| H | $CH_3$ | F | $-S-CH_2-C\equiv CH$ | O |
| H | $CH_3$ | F | $-S-C_2H_5$ | O |
| H | $CH_3$ | F | $-O-i-C_3H_7$ | O |
| H | $CH_3$ | F | $-CH_2-CN$ | O |
| H | $CH_3$ | F | $-O-CH(CH_3)-COO-CH_2-C\equiv CH$ | O |
| H | $CH_3$ | F | 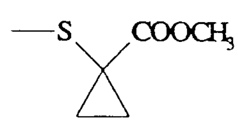 | O |

| $R^1$ | $R^2$ | $R^3$ | $R^4$ | X |
|---|---|---|---|---|
| H | $CH_3$ | F | 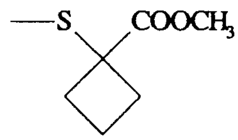 | O |
| H | $CH_3$ | Cl | $-O-CH_2-C{\equiv}CH$ | O |
| H | $CH_3$ | Cl | $-O-C_2H_5$ | O |
| H | $CH_3$ | Cl | $-O-CH_3$ | O |
| H | $CH_3$ | Cl | $-S-CH_3$ | O |
| H | $CH_3$ | Cl | $-O-CH_2-CF_3$ | O |
| H | $CH_3$ | Cl | $-O-CH(CH_2F)_2$ | O |
| H | $CH_3$ | Cl | $-O-(CH_2-CH_2-O)_2-CH_3$ | O |
| H | $CH_3$ | Cl | $-O-CH_2-CH_2-O-C_2H_5$ | O |
| H | $CH_3$ | Cl | $-O-CH(CH_3)-COOC_2H_5$ | O |
| H | $CH_3$ | Cl | $-O-CH_2-COO-n-C_4H_9$ | O |
| H | $CH_3$ | Cl | $-O-CH_2-CH=CH_2$ | O |
| H | $CH_3$ | Cl | $-O-CH(CH_3)-C{\equiv}CH$ | O |
| H | $CH_3$ | Cl | $-S-CH_2-COOCH_3$ | O |
| H | $CH_3$ | Cl | $-S-CH_2-C{\equiv}CH$ | O |
| H | $CH_3$ | Cl | $-S-C_2H_5$ | O |

| $R^1$ | $R^2$ | $R^3$ | $R^4$ | X |
|---|---|---|---|---|
| H | $CH_3$ | Cl | $-O-i-C_3H_7$ | O |
| H | $CH_3$ | Cl | $-CH_2-CN$ | O |
| H | $CH_3$ | Cl | $-O-CH(CH_3)-COO-CH_2-C\equiv CH$ | O |
| H | $CH_3$ | Cl | 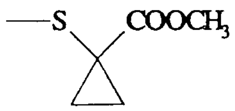 | O |
| H | $CH_3$ | Cl | 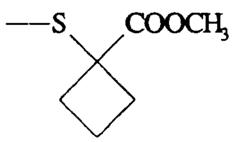 | O |

Verwendet man beispielsweise 3-Trifluormethyl-4-methoxy-1,2,4-triazolin-5-on und 2,4,5-Trifluorbenzonitril als Ausgangsstoffe, so läßt sich der Reaktionsablauf des erfindungsgemäßen Verfahrens (a) durch das folgende Formelschema darstellen:

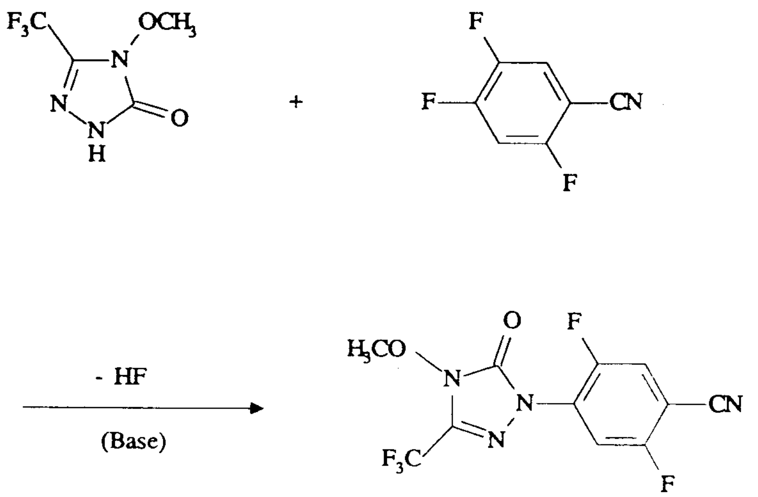


Verwendet man beispielsweise 1-(4-Cyano-2,5-difluorphenyl)-4-methoxy-3-trifluormethyl-1,2,4-triazolin-5-on und 3-Butin-2-ol als Ausgangsstoffe, so läßt sich der Reaktionsablauf des erfindungsgemäßen Verfahrens (b) durch das folgende Formelschema darstellen:

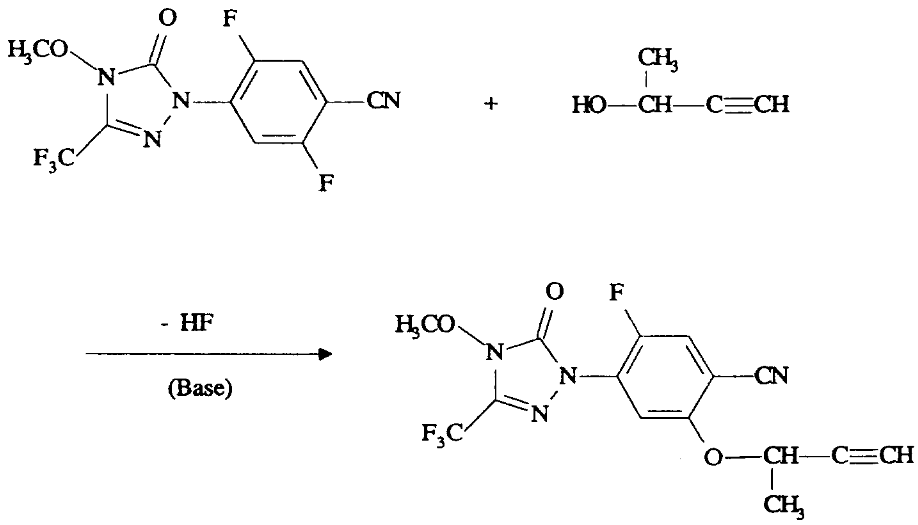


Verwendet man beispielsweise 1-(4-Cyano-2,5-difluophenyl)-5-trifluormethyl-(4H)-1,2,4-triazolin-5-on und Dichlorfluormethylsulfenylchlorid als Ausgangsstoffe, so läßt sich der Reaktionsablauf des erfindungsgemäßen Verfahrens (c) durch das folgende Formelschema darstellen:

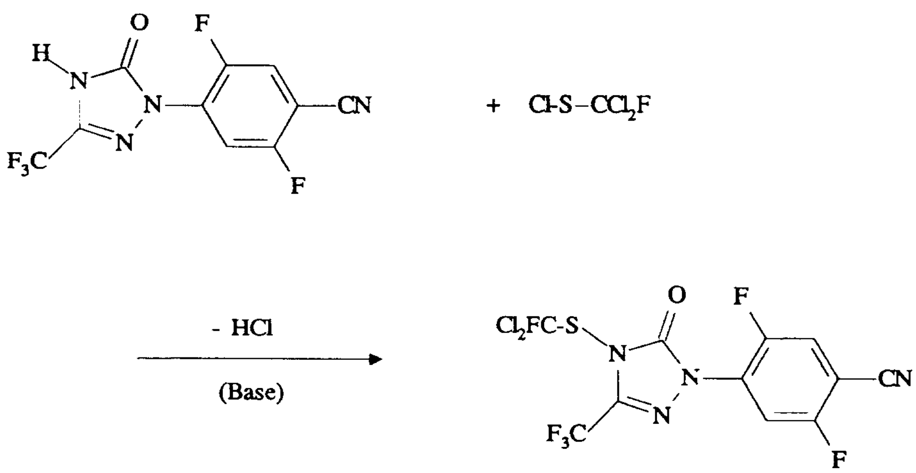


Die zur Durchführung des erfindungsgemäßen Verfahrens (a) als Ausgangsstoffe benötigten 1H-Triazolinone sind durch die Formel (II) allgemein definiert. In dieser Formel (II) stehen $R^1$, $R^2$ und X vorzugsweise und besonders bevorzugt für diejenigen Reste, die bereits im Zusammenhang mit der Beschreibung der erfindungsgemäßen Verbindungen der Formel (1) als bevorzugt und besonders bevorzugt für diese Substituenten genannt wurden.

Die 1H-Triazolinone der Formel (II) sind bekannt oder erhältlich in Analogie zu bekannten Verfahren (vergl. z.B. EP 422 469; Arch. Pharm 320, 1167-1173 [1987]; JP 60016978; Arch. Pharm. 301, 827-829 [1968]; DE 41 31 842).

Die zur Durchführung des erfindungsgemäßen Verfahrens (a) weiterhin als Ausgangsstoffe benötigten Halogenbenzol-Derivate sind durch die Formel (III) allgemein definiert. In dieser Formel (III) stehen $R^3$, $R^4$ und $R^5$ vorzugsweise und besonders bevorzugt für diejenigen Reste, die bereits im Zusammenhang mit der Beschreibung der erfindungsgemäßen Verbindungen der Formel (I) als bevorzugt und besonders bevorzugt für diese Substituenten genannt wurden. Hal steht vorzugsweise für Fluor, Chlor oder Brom, insbesondere für Fluor oder Chlor.

Die Halogenbenzol-Derivate der Formel (III) sind bekannt. oder erhältlich in Analogie zu bekannten Verfahren (vergl. z.B. EP 191 181; EP 441 004; EP 431 373).

Die zur Durchführung des erfindungsgemäßen Verfahren (b) als Ausgangsprodukte benötigten substituierten Triazolinone sind durch die Formel (Ia) allgemein definiert. In dieser Formel (Ia) stehen $R^1$, $R^2$, $R^3$, $R^5$

und X vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden. $R^8$ steht vorzugsweise für Fluor, Chlor oder Brom, insbesondere für Fluor oder Chlor.

Die substituierten Triazolinone der Formel (Ia) sind erfindungsgemäße Verbindungen und erhältlich mit Hilfe der erfindungsgemäßen Verfahren (a) und/oder (c).

Die zur Durchführung des erfindungsgemäßen Verfahren (b) weiterhin als Ausgangsprodukte benötigten Nukleophile sind durch die Formel (IV) allgemein definiert. In dieser Formel (IV) steht Z vorzugsweise für Sauerstoff, Schwefel oder für den Rest -N($R^7$)-. $R^9$ steht vorzugsweise und besonders bevorzugt für diejenigen Reste, die bereits im Zusammenhang mit der Beschreibung der erfindungsgemäßen Stoffe der Formel (I) als bevorzugt und besonders bevorzugt für den Substituenten $R^6$ genannt wurden mit Ausnahme des Wasserstoff-Restes. $R^7$ steht vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diesen Substituenten genannt wurden. Die Nukleophile der Formel (IV) sind allgemein bekannte Verbindungen der organischen Chemie.

Die zur Durchführung des erfindungsgemäßen Verfahren (c) als Edukte benötigten substituierten Triazolinone sind durch die Formel (V) allgemein definiert. In dieser Formel (Ib) stehen $R^1$, $R^3$, $R^4$ und $R^5$ vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diese Substituenten genannt wurden.

Die substituierten Triazolinone der Formel (V) sind bekannt oder erhältlich in Analogie zu bekannten Verfahren (vergl. z.B. DE 38 39 480; US 4.894.084; Angew. Chemie 85, 447-448 [1973]; Synth. Commun 16, 163-167 [1986]; Liebigs Ann. Chem. 722, 29-37 [1969]; J. Indian Chem. Soc. 7, 899-901 [1930]).

Die zur Durchführung des erfindungsgemäßen Verfahrens (c) weiterhin als Edukte benötigten Sulfenylierungsmittel sind durch die Formel (VI) allgemein definiert. In dieser Formel (VI) steht $R^2$ vorzugsweise und besonders bevorzugt für diejenigen Reste, die bereits im Zusammenhang mit der Beschreibung der erfindungsgemäßen Stoffe der Formel (I) als bevorzugt und besonders bevorzugt für diesen Substituenten genannt wurden. Hal steht vorzugsweise für Fluor, Chlor, Brom oder Iod , insbesondere für Chlor.

Die Sulfenylierungsmittel der Formel (V) sind allgemein bekannte Verbindungen der organischen Chemie.

Als Verdünnungsmittel zur Durchführung des erfindungsgemäßen Verfahrens (a) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid oder Ester, wie Essigsäuremethylester oder Essigsäureethylester oder Alkohole wie Methanol, Ethanol, Propanol, Ethylenglykolmonomethylether oder Diethylenglykolmonomethylether.

Das erfindungsgemäße Verfahren (a) wird vorzugsweise in Gegenwart eines geeigneten Reaktionshilfsmittels durchgeführt. Als solche kommen alle üblichen anorganischen oder organischen Basen oder Fluoride infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Cäsiumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, Natriumfluorid, Kaliumfluorid sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im allgemeinen arbeitet man bei Temperaturen zwischen 0 °C und +180 °C, vorzugsweise bei Temperaturen zwischen +20 °C und +120 °C.

Das erfindungsgemäße Verfahren (a) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (a) setzt man pro Mol 1H-Triazolinon der Formel (II) im allgemeinen 1,0 bis 3,0 Mol, vorzugsweise 1,0 bis 1,5 Mol Halogenbenzol-Derivat der Formel (III) und gegebenenfalls 1,0 bis 3,0 Mol, vorzugsweise 1,0 bis 1,5 Mol Base als Reaktionshilfsmittel ein. Die Reaktionsdurchführung, Aufarbeitung und Isolierung der Reaktionsprodukte erfolgt nach bekannten Verfahren (vergl. hierzu auch die Herstellungsbeispiele).

Als Verdünnungsmittel zur Durchführung des erfindungsgemäßen Verfahrens (b) kommen inerte organische Lösungsmittel infrage. Vorzugsweise verwendet man die bei der Beschreibung der Durchführung des erfindungsgemäßen Verfahrens (a) aufgezählten Lösungsmittel.

Das erfindungsgemäße Verfahren (b) wird vorzugsweise in Gegenwart eines geeigneten Reaktionshilfsmittels durchgeführt. Als solche kommen alle üblichen anorganischen oder organischen Basen oder Fluoride infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Cäsiumcarbonat, Kaliumhydrogencarbonat, Natriumhydrogencarbonat oder Ammoniumcarbonat, Natriumfluorid, Kaliumfluorid sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und +180 °C, vorzugsweise bei Temperaturen zwischen +20 °C und +120 °C.

Das erfindungsgemäße Verfahren (b) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (b) setzt man pro Mol substituiertem Triazolinon der Formel (Ia) im allgemeinen 1,0 bis 3,0 Mol, vorzugsweise 1,0 bis 1,5 Mol Nukleophil der Formel (IV) und gegebenenfalls 0,1 bis 3,0 Mol, vorzugsweise 1,0 bis 1,5 Mol Base als Reaktionshilfsmittel ein.

Die Reaktionsdurchführung, Aufarbeitung und Isolierung der Reaktionsprodukte erfolgt nach bekannten Verfahren (vergl. hierzu auch die Herstellungsbeispiele).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl-oder -diethylether; Ketone, wie Aceton, Butanon oder Methyl-isobutylketon; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Säuren wie Essigsäure.

Das erfindungsgemäße Verfahren (c) wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali-oder Alkalimetallhydroxide, wie Natriumhydroxid, Calciumhydroxid, Kaliumhydroxid oder auch Ammoniumhydroxid, Alkalimetallcarbonate, wie Natriumcarbonat, Kaliumcarbonat, Cäsiumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (c) in einem größeren Bereich variiert werden. Im allgemeinen arbeitet man bei Temperaturen zwischen -20 °C und +120 °C, vorzugsweise bei Temperaturen zwischen 0 °C und +80 °C.

Das erfindungsgemäße Verfahren (d) wird üblicherweise unter Normaldruck durchgeführt. Es ist jedoch auch möglich unter erhöhtem oder vermindertem Druck zu arbeiten.

Zur Durchführung des erfindungsgemäßen Verfahrens (d) setzt man pro Mol substituiertem Triazolinon der Formel (V) im allgemeinen 1,0 bis 3,0 Mol, vorzugsweise 1,0 bis 2,0 Mol Sulfenylierungsmittel der Formel (VI) und gegebenenfalls 1,0 bis 3,0 Mol, vorzugsweise 1,0 bis 2,0 Mol Base als Reaktionshilfsmittel ein.

Die Reaktionsdurchführung, Aufarbeitung und Isolierung der Reaktionsprodukte erfolgt nach bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen mit Hilfe der Protonen-Kernresonanzspekroskopie ($^1$H-NMR).

Die erfindungsgemäßen Wirkstoffe können als Defoliants, Desiccants, Krautabtötungsmittel und insbesondere als Unkrautvernichtungsmittel verwendet werden.Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Zitrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur selektiven Bekämpfung von mono- und dikotylen Unkräutern in dikotylen Kulturen wie beispielsweise Soja oder Baumwolle einsetzen.

Daneben besitzen die erfindungsgemäßen Wirkstoffe auch fungizide Eigenschaften und lassen sich in entsprechenden Aufwandmengen beispielsweise auch zur Bekämpfung von Getreidekrankheiten, wie beispielsweise gegen den Erreger des echten Getreidemehltaues (Erysiphe graminis) oder zur Bekämpfung von Reiskrankheiten, wie beispielsweise gegen den Erreger der Reisfleckenkrankheit (Pyricularia oryzae) einsetzen.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Fälle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser.

Als feste Trägerstoffe kommen infrage: z.B. Ammoniumsalze und natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol,

Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba oder Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen; Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro Hektar.

Die Herstellung und die Verwendung der erfindungsgemäßen Wirkstoffe geht aus den nachfolgenden Beispielen hervor.

Herstellungsbeispiele:

Beipiel 1:

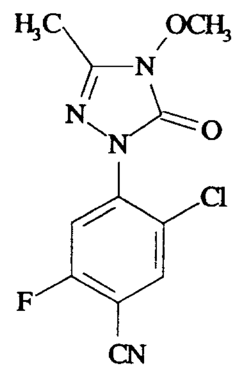

(Verfahren a)

Zu 5,2 g (0,04 Mol) 4-Methoxy-3-methyl-1H-1,2,4-triazolin-5-on (vergl. z.B. EP 422 469) und 5,5 g (0,04 Mol) Kaliumcarbonat in 50 ml Dimethylsulfoxid gibt man bei Raumtemperatur 7,0 g (0,04 Mol) 5-Chlor-2,4-difluorbenzonitril und rührt anschließend 16 Stunden bei Raumtemperatur. Zur Aufarbeitung wird die Reaktionsmischung im Vakuum eingeengt, der Rückstand in Dichlormethan aufgenommen und nacheinander mit verdünnter Salzsäure und Wasser gewaschen, über Magnesiumsulfat getrocknet und im Vakuum vom Lösungsmittel befreit.

Man erhält 8,5 g (75 % der Theorie) 1-(2-Chlor-4-cyano-5-fluor-phenyl)-4-methoxy-3-methyl-1,2,4-triazolin-5-on vom Schmelzpunkt 102 °C.

Herstellung der Ausgangsverbindung:

Beispiel III-1:

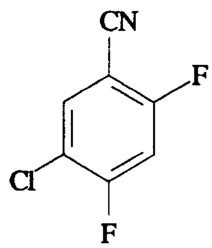

Zu 250 g (4,31 Mol) Kaliumfluorid in 400 ml destilliertem Tetramethylensulfon gibt man unter Rühren bei Raumtemperatur 220 g (1,06 Mol) 2,4,5-Trichlorbenzonitril (vergl. z.B. EP 441 004) und rührt anschließend 10 Stunden bei 195 °C bis 200 °C. Zur Aufarbeitung wird abgekühlt, 500 ml Wasser zugegeben und die Mischung einer Wasserdampfdestillation unterzogen. Der organische Anteil wird in Dichlormethan aufgenommen, über Natriumsulfat getrocknet, im Vakuum eingeengt und destilliert

Man erhält 108 g (58 % der Theorie) 2,4-Difluor-5-chlorbenzonitril vom Siedepunkt 105-107 °C bei 30 mbar und vom Schmelzpunkt 48-50 °C.

Beispiel 2:

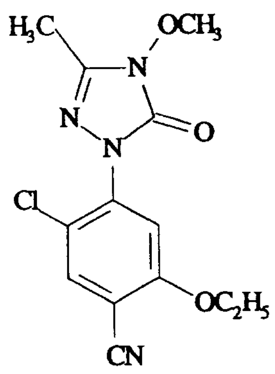

Verfahren (b)

Zu einer Lösung von 0,012 Mol Natriumethylat in 10 ml Ethanol gibt man bei Raumtemperatur unter Rühren 2,8 g (0,01 Mol) 1-(2-Chlor-4-cyano-5-fluor-phenyl)-4-methoxy-3-methyl-1,2,4-triazolin-5-on in 10 ml trockenem Ethanol gelöst und rührt anschließend 16 Stunden bei Raumtemperatur. Zur Aufarbeitung wird das Reaktionsgemisch im Vakuum eingeengt, der Rückstand in 30 ml Dichlormethan aufgenommen, nacheinander mit verdünnter Salzsäure und Wasser gewaschen, über Natriumsulfat getrocknet und im Vakuum vom Lösungsmittel befreit. Der Rückstand läßt sich durch Chromatographie an Kieselgel (Laufmittel: Dichlormethan/Methanol 40:1) reinigen.

Man erhält 0,8 g (25 % der Theorie) 1-(2-Chlor-4-cyano-5-ethoxy-phenyl)-4-methoxy-3-methyl-1,2,4-triazolin-5-on vom Schmelzpunkt 115 °C.

Beispiel 3:

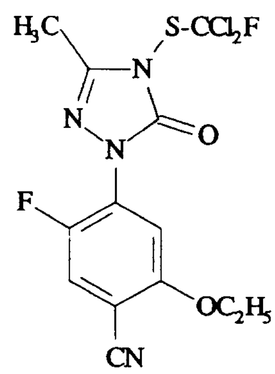

Verfahren (c)

Zu einer Lösung von 2,6 g (0,01 Mol) 1-(2-Fluor-4-cyano-5-ethoxy-phenyl)-3-methyl-4H-1,2,4-triazolin-5-on in 80 ml Tetrachlorkohlenstoff gibt man unter Rühren bei 0 °C bis 5 °C nacheinander 4,2 g (0,025 Mol) Dichlorfluormethylsulfenylchlorid und 2,5 g (0,025 Mol) Triethylamin und erwärmt anschließend für 12 Stunden auf Rückflußtemperatur. Zur Aufarbeitung wird die Reaktionsmischung mit Wasser gewaschen, über Natriumsulfat getrocknet und im Vakuum eingeengt. Der Rückstand läßt sich durch Chromatographie an Kieselgel (Laufmittel: Dichlormethan/Methanol 100:1) reinigen.

Man erhält 1,3 g (33 % der Theorie) 1-(2-Fluor-4-cyano-5-ethoxy-phenyl)-3-methyl-4-dichlorfluormethylsulfenyl-1,2,4-triazolin-5-on vom Schmelzpunkt 185 °C.

Herstellung der Ausgangsverbindung:

Beispiel V-1:

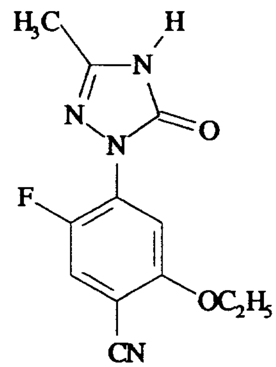

Zu einer Lösung von 0,07 Mol Natriumethylat in 40 ml Ethanol gibt man bei Raumtemperatur unter Rühren 5,4 g (0,023 Mol) 1-(2,5-Difluor-4-cyano-phenyl)-3-methyl-4H-1,2,4-triazolin-5-on (vergl. z.B. DE 38 39 480) in 40 ml trockenem Ethanol gelöst, versetzt die Mischung mit 100 ml N-Methylpyrrolidon, destilliert dann so viel Ethanol ab, bis die Innentemperatur des Reaktionsgemisches 130 °C erreicht und rührt anschließend weitere 7 Stunden bei dieser Temperatur. Zur Aufarbeitung wird das Reaktionsgemisch im Vakuum eingeengt, der Rückstand in Dichlormethan aufgenommen, nacheinander mit verdünnter Salzsäure und Wasser gewaschen, über Natriumsulfat getrocknet und im Vakuum vom Lösungsmittel befreit.

Man erhält 5,3 g (88 % der Theorie) 1-(2-Fluor-4-cyano-5-ethoxy-phenyl)-3-methyl-4H-1,2,4-triazolin-5-on vom Schmelzpunkt 204 °C.

In entsprechender Weise und gemäß den allgemeinen Angaben zur Herstellung erhält man die folgenden substituierten Triazolinone der allgemeinen Formel (I):

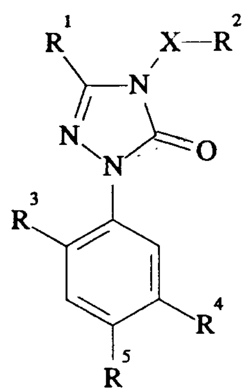

(I)

| Bsp. Nr. | 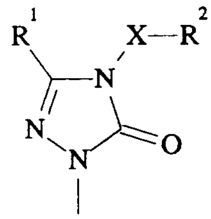 | $R^3$ | $R^4$ | $R^5$ | physikalische Eigenschaften |
|---|---|---|---|---|---|
| 4 | 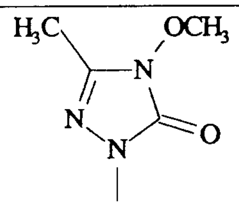 | F | $-S-n-C_4H_9$ | CN | Fp. 115°C |
| 5 | 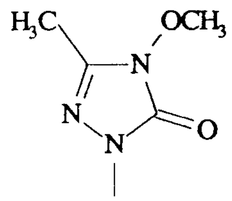 | F | H | CN | Fp. 156°C |
| 6 | 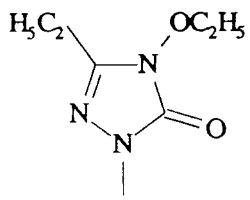 | F | H | CN | Fp. 78°C |

| Bsp. Nr. | 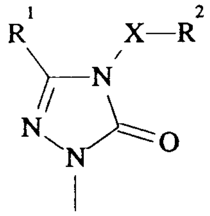 | $R^3$ | $R^4$ | $R^5$ | physikalische Eigenschaften |
|---|---|---|---|---|---|
| 7 | 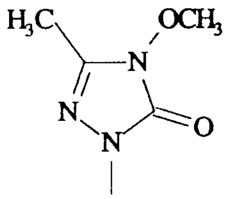 | F | $-S-CH_2-COOC_2H_5$ | CN | Fp. 65°C |
| 8 | 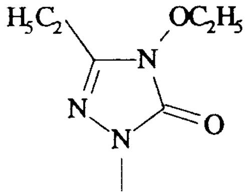 | H | F | CN | Fp. 114°C |
| 9 | 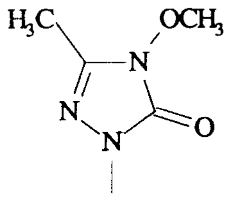 | H | F | CN | Fp. 185°C |
| 10 | 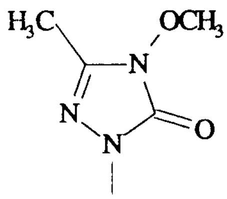 | F | 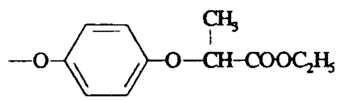 | CN | $^1$H-NMR*): 7,48 (d, 1H) |
| 11 | 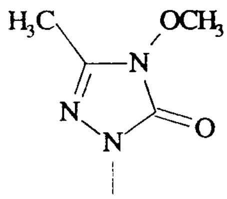 | F | $-O-CH_2-C_6H_5$ | CN | Fp. 143°C |
| 12 | 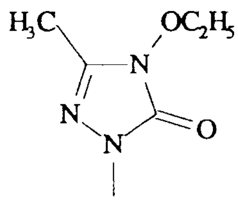 | F | F | CN | Fp. 89°C |

| Bsp. Nr. | 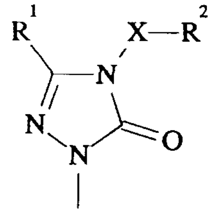 | $R^3$ | $R^4$ | $R^5$ | physikalische Eigenschaften |
|---|---|---|---|---|---|
| 13 | 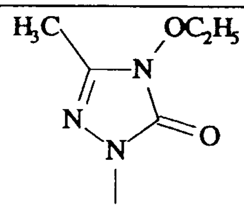 | F | H | CN | Fp. 138°C |
| 14 | 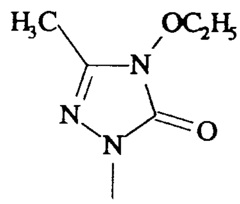 | F | $-O-CH_3$ | CN | Fp. 97°C |
| 15 | 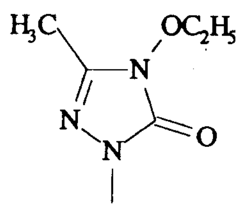 | F | $-O-C_2H_5$ | CN | Fp. 80°C |
| 16 | 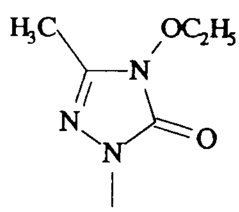 | F | $-O-CH(CH_3)-C{\equiv}CH$ | CN | |
| 17 | 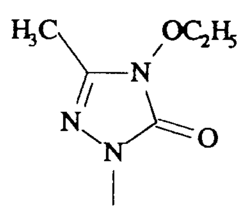 | F | 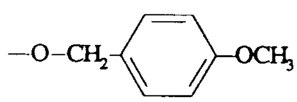 | CN | $^1H$-NMR*): 5,12 (s, 2H) |
| 18 | 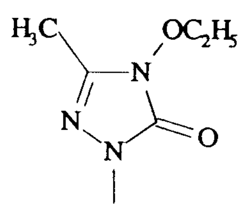 | F | $-(O-CH_2-CH_2)_2-OCH_3$ | CN | Fp. 50°C |

| Bsp. Nr. | 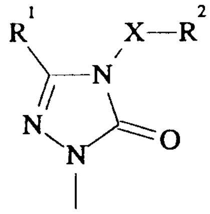 | $R^3$ | $R^4$ | $R^5$ | physikalische Eigenschaften |
|---|---|---|---|---|---|
| 19 | 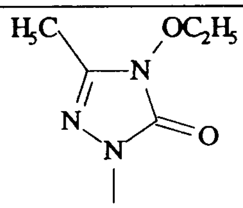 | F | $-S-C_2H_5$ | CN | Fp. 78°C |
| 20 | 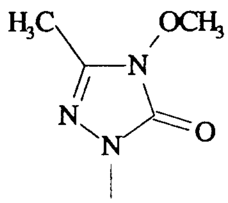 | F | $-O-CH_2-CH_2-OCH_3$ | CN | Fp. 114°C |
| 21 | 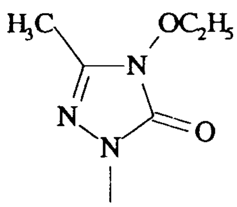 | F | $-S-CH_3$ | CN | |
| 22 | 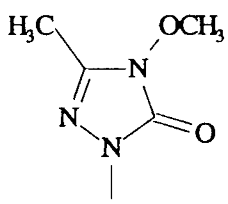 | F | $-S-CH_3$ | CN | |
| 23 | 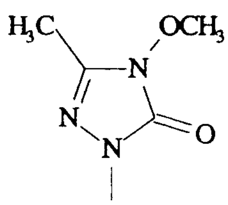 | H | CN | CN | Fp. 174-175°C |
| 24 | 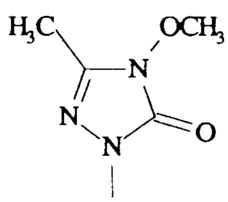 | F | Cl | CN | Fp. 167°C |

| Bsp. Nr. | 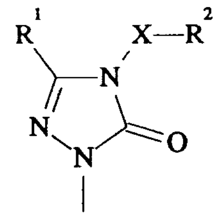 | $R^3$ | $R^4$ | $R^5$ | physikalische Eigenschaften |
|---|---|---|---|---|---|
| 25 | 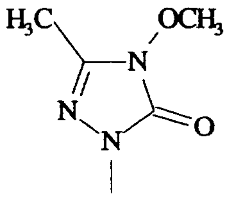 | F | F | CN | Fp. 107°C |
| 26 | 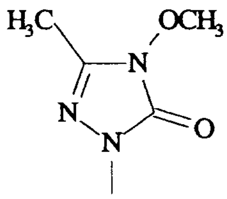 | F | $-O-CH_3$ | CN | Fp. 160°C |
| 27 | 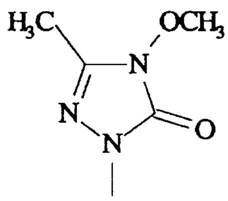 | F | $-O-CH(CH_3)-C{\equiv}CH$ | CN | Fp. 118°C |
| 28 | 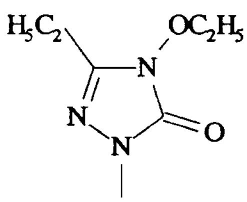 | F | F | CN | Fp. 40°C |
| 29 | 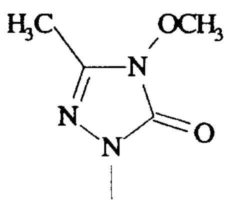 | F | $-O-C_2H_5$ | CN | Fp. 114°C |
| 30 | 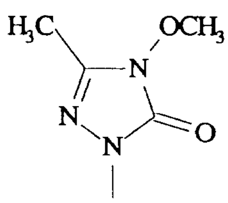 | F | $-S-C_2H_5$ | CN | Fp. 104°C |

| Bsp. Nr. | 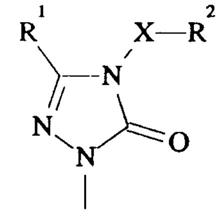 | $R^3$ | $R^4$ | $R^5$ | physikalische Eigenschaften |
|---|---|---|---|---|---|
| 31 | 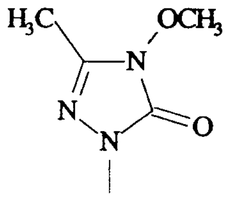 | Cl | $-O-n-C_3H_7$ | CN | Fp. 132°C |
| 32 | 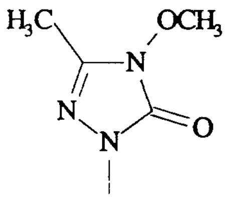 | Cl | $-(O-CH_2-CH_2)_2-OCH_3$ | CN | Fp. 61°C |
| 33 | 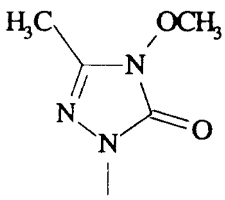 | Cl | $-S-n-C_4H_9$ | CN | Fp. 104°C |
| 34 | 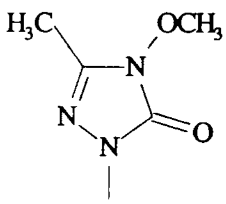 | Cl | $-S-C_2H_5$ | CN | Fp. 106°C |
| 35 | 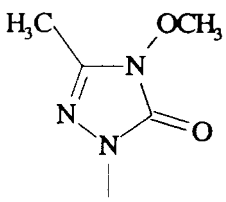 | Cl | $-O-CH(CH_3)-C{\equiv}CH$ | CN | $^1H$-NMR*): 2,59 (d, 1H) |
| 36 | 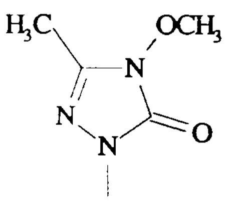 | Cl | $-S-CH_2-COOC_2H_5$ | CN | |

| Bsp. Nr. | 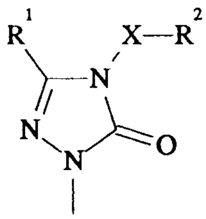 | $R^3$ | $R^4$ | $R^5$ | physikalische Eigenschaften |
|---|---|---|---|---|---|
| 37 | 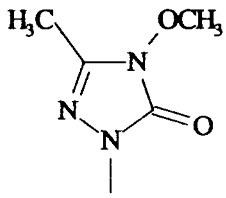 | Cl | 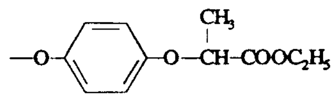 | CN | $^1$H-NMR*): 4,72 (q, 1H) |
| 38 | 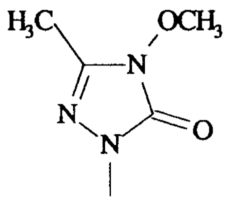 | H | $-O-CH(CH_3)-C{\equiv}CH$ | CN | |
| 39 | 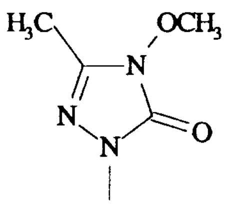 | H | $-(O-CH_2-CH_2)_2-OCH_3$ | CN | |
| 40 | 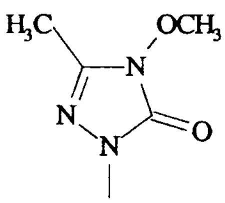 | Cl | $-S-CH_3$ | CN | Fp. 175°C |
| 41 | 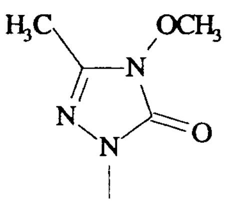 | F | $-O-i-C_4H_9$ | CN | |
| 42 | 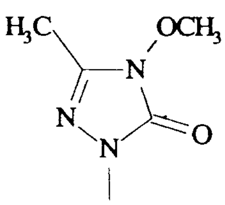 | F | $-(O-CH_2-CH_2)_2-OCH_3$ | CN | Fp. 68°C |

| Bsp. Nr. | 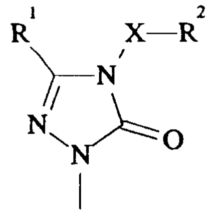 | $R^3$ | $R^4$ | $R^5$ | physikalische Eigenschaften |
|---|---|---|---|---|---|
| 43 | 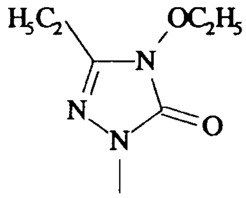 | F | $-O-CH_3$ | CN | Fp. 66°C |
| 44 | 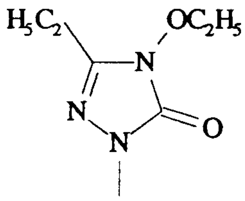 | F | $-O-C_2H_5$ | CN | $^1H$-NMR*): 7,41 (d, 1H) |
| 45 | 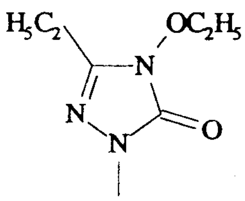 | F | $-O-i-C_3H_7$ | CN | Fp. 105°C |
| 46 | 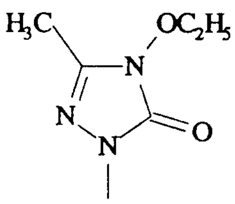 | Cl | $-S-C_2H_5$ | CN | Fp. 116-118°C |
| 47 | 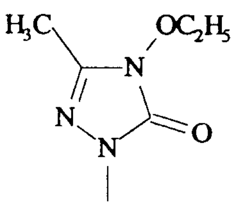 | Cl | $-O-CH(CH_3)-C{\equiv}CH$ | CN | Fp. 125-126°C |
| 48 | 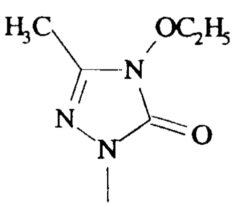 | Cl | $-O-CH_2-Si(CH_3)_3$ | CN | Fp. 103-105°C |

| Bsp. Nr. | 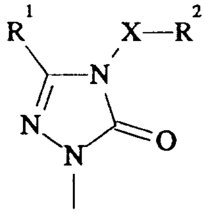 | $R^3$ | $R^4$ | $R^5$ | physikalische Eigenschaften |
|---|---|---|---|---|---|
| 49 | 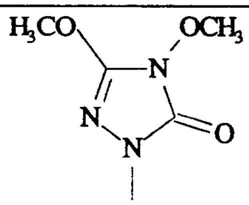 | F | F | CN | Fp. 132°C |
| 50 | 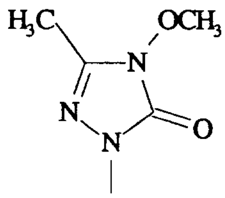 | F | F | $NO_2$ | Fp. 150-151°C |
| 51 | 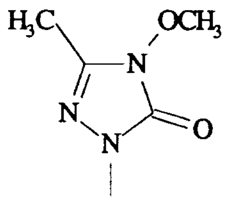 | F | $-O-CH(CH_3)-C\equiv CH$ | $NO_2$ | Fp. 118-120°C |
| 52 | 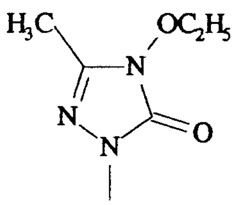 | Cl | Cl | $NO_2$ | Fp. 158-160°C |
| 53 | 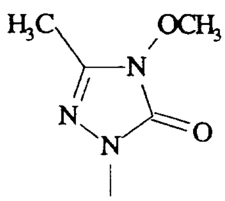 | Cl | H | $NO_2$ | Fp. 185-186°C |
| 54 | 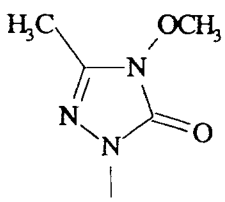 | Cl | F | CN | Fp. 126-128°C |

| Bsp. Nr. | 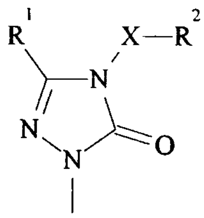 | $R^3$ | $R^4$ | $R^5$ | physikalische Eigenschaften |
|---|---|---|---|---|---|
| 55 | 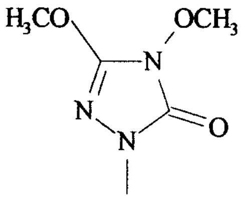 | F | 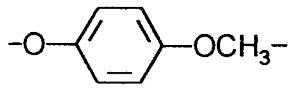 | CN | Fp. 180 C |
| 56 | 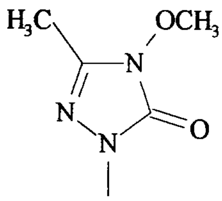 | F | 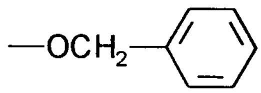 | $NO_2$ | Fp. 112 C |
| 57 | 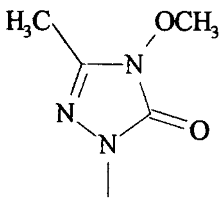 | F | 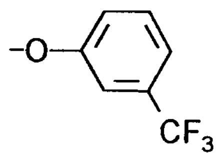 | $NO_2$ | Fp. 112 C |
| 58 | 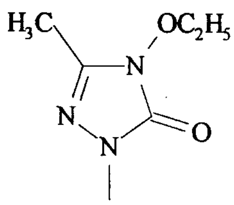 | Cl | -SH | $NO_2$ | Fp. 187 C |
| 59 | 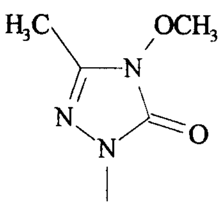 | Cl | 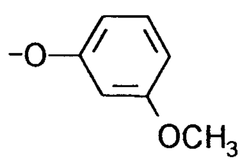 | $NO_2$ | Fp. 127 C |
| 60 | 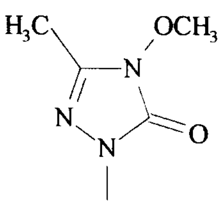 | Cl | 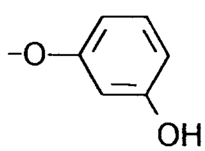 | CN | |

| Bsp. Nr. | 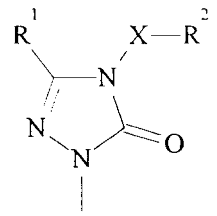 | $R^3$ | $R^4$ | $R^5$ | physikalische Eigenschaften |
|---|---|---|---|---|---|
| 61 | 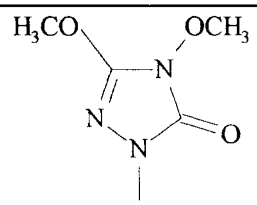 | F | 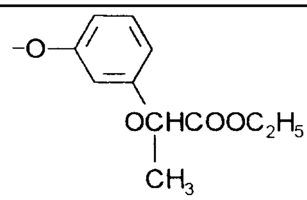 | CN | |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Anwendungsbeispiele:

In dem folgenden Anwendungsbeispiel wurde die nachstehend aufgeführte Verbindung als Vergleichssubstanz eingesetzt:

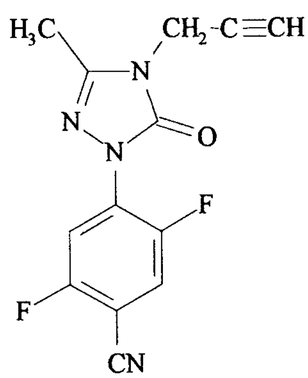

(A)

3-Methyl-4-propargyl-1-(2,5-difluor-4-cyano-phenyl)-1,2,4-triazolin-5-on
(bekannt aus DE 38 39 480)

Beispiel A:

**Pre-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man ein Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffes pro Flächeneinheit. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)

100 % = totale Vernichtung

Eine deutliche Überlegenheit in der Wirksamkeit gegenüber dem Stand der Technik zeigen in diesem Test z.B. die Verbindungen gemäß den Herstellungsbeispielen 24, 25, 26, 27, 29 und 42.

**Patentansprüche**

**1.** Substituierte Triazolinone der allgemeinen Formel (I),

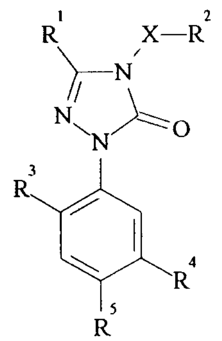

(I)

in welcher

| | |
|---|---|
| $R^1$ | für Wasserstoff, Alkyl, Alkoxy, Halogenalkyl oder Halogenalkoxy steht, |
| $R^2$ | für Wasserstoff, Alkyl oder Halogenalkyl steht, |
| $R^3$ | für Wasserstoff oder Halogen steht, |
| $R^4$ | für Wasserstoff, Cyano, Halogen oder für einen Rest der Formel $-O-R^6$, $-S-R^6$, $-C(O)-O-R^6$, $-C(O)-S-R^6$, $-NR^6R^7$ oder $-C(O)-NR^6R^7$ steht, |
| $R^5$ | für Cyano oder Nitro steht und |
| X | für Sauerstoff oder Schwefel steht, wobei |
| $R^6$ und $R^7$ | unabhängig voneinander jeweils für Wasserstoff oder für jeweils gegebenenfalls substituiertes Alkyl, Alkenyl, Alkinyl, Cycloalkyl, Cycloalkylalkyl, Cycloalkoxycarbonyl, Aryl oder Arylalkyl stehen. |

**2.** Substituierte Triazolinone der allgemeinen Formel (I) gemäß Anspruch 1, dadurch gekennzeichnet, daß

| | |
|---|---|
| $R^1$ | für Wasserstoff oder für jeweils geradkettiges oder verzweigtes Alkyl, Alkoxy, Halogenalkyl oder Halogenalkoxy mit jeweils 1 bis 8 Kohlenstoffatomen und gegebenenfalls 1 bis 17 gleichen oder verschiedenen Halogenatomen - insbesondere Fluor, Chlor, Brom oder Iod - steht, |
| $R^2$ | für Wasserstoff oder für jeweils geradkettiges oder verzweigtes Alkyl oder Halogenalkyl mit jeweils 1 bis 8 Kohlenstoffatomen und gegebenenfalls 1 bis 17 gleichen oder verschiedenen Halogenatomen - insbesondere Fluor, Chlor, Brom oder Iod - steht, |
| $R^3$ | für Wasserstoff Fluor, Chlor, Brom oder Iod steht, |
| $R^4$ | für Wasserstoff Cyano, Fluor, Chlor, Brom, Iod oder für einen Rest der Formel $-O-R^6$, $-S-R^6$, $-C(O)-O-R^6$, $-C(O)-S-R^6$, $-NR^6R^7$ oder $-C(O)-NR^6R^7$ steht, |
| $R^5$ | für Cyano oder Nitro steht und |
| X | für Sauerstoff oder Schwefel steht, wobei |
| $R^6$ und $R^7$ | unabhängig voneinander jeweils für Wasserstoff oder für gegebenenfalls einfach oder mehrfach, gleich oder verschieden substituiertes, geradkettiges oder verzweigtes Alkyl mit 1 bis 8 Kohlenstoffatomen stehen, wobei als Substituenten infrage kommen: Halogen - insbesondere Fluor, Chlor, Brom und/oder Iod - , Cyano, Carboxyl, Carbamoyl, jeweils geradkettiges oder verzweigtes Alkoxy, Alkoxyalkoxy, Halogenal- |

koxy (wobei Halogen für Fluor, Chlor, Brom und/oder Iod steht), Alkylthio, Alkylsulfinyl, Alkylsulfonyl, Alkylcarbonyl, Alkoxycarbonyl, Alkenyloxycarbonyl, Alkinyloxycarbonyl, Alkoxyalkoxycarbonyl, N-Alkylaminocarbonyl, N,N-Dialkylaminocarbonyl, Trialkylsilyl oder Alkylsulfonylaminocarbonyl mit jeweils bis zu 8 Kohlenstoffatomen in den einzelnen Alkyl- bzw. Alkenyl-oder Alkinylteilen oder Heterocyclyl, wobei als Heterocyclylrest ein fünf-bis siebengliedriger, gegebenenfalls benzannellierter, gesättigter oder ungesättigter Heterocyclus mit 1 bis 3 gleichen oder verschiedenen Heteroatomen - insbesondere Stickstoff Sauerstoff und/ oder Schwefel - steht;

$R^6$ und $R^7$ außerdem für jeweils gegebenenfalls einfach oder mehrfach, gleich oder verschieden durch Fluor, Chlor, Brom und/oder Iod substituiertes Alkenyl oder Alkinyl mit jeweils 2 bis 8 Kohlenstoffatomen stehen;

$R^6$ und $R^7$ außerdem für jeweils gegebenenfalls im Cycloalkylteil einfach oder mehrfach, gleich oder verschieden durch Fluor, Chlor, Brom und/oder Iod und/oder jeweils geradkettiges oder verzweigtes Alkyl und/oder Alkoxy und/oder Alkoxycarbonyl mit jeweils 1 bis 4 Kohlenstoffatomen substituiertes Cycloalkyl, Cycloalkylalkyl oder Cycloalkyloxycarbonyl mit jeweils 3 bis 8 Kohlenstoffatomen im Cycloalkylteil und gegebenenfalls 1 bis 4 Kohlenstoffatomen im geradkettigen oder verzweigten Alkylteil stehen, oder

$R^6$ und $R^7$ für jeweils gegebenenfalls im Arylteil einfach oder mehrfach, gleich oder verschieden substituiertes Arylalkyl oder Aryl mit jeweils 6 bis 10 Kohlenstoffatomen im Arylteil und gegebenenfalls 1 bis 4 Kohlenstoffatomen im geradkettigen oder verzweigten Alkylteil stehen, wobei als Arylsubstituenten jeweils infrage kommen:
Halogen, Cyano, Nitro, Hydroxy, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, geradkettiges oder verzweigtes Alkoxycarbonylalkyloxy mit 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 13 gleichen oder verschiedenen Halogenatomen substituiertes Phenyl, wobei Halogen jeweils Fluor, Chlor, Brom und/oder Iod bedeutet.

**3.** Substituierte Triazolinone der allgemeinen Formel (I) (gemäß Anspruch 1, dadurch gekennzeichnet, daß

$R^1$ für Wasserstoff oder für jeweils geradkettiges oder verzweigtes Alkyl, Alkoxy, Halogenalkyl oder Halogenalkoxy mit jeweils 1 bis 6 Kohlenstoffatomen und gegebenenfalls 1 bis 13 gleichen oder verschiedenen Halogenatomen - insbesondere Fluor, Chlor oder Brom - steht,

$R^2$ für Wasserstoff oder für jeweils geradkettiges oder verzweigtes Alkyl oder Halogenalkyl mit jeweils 1 bis 6 Kohlenstoffatomen und gegebenenfalls 1 bis 13 gleichen oder verschiedenen Halogenatomen - insbesondere Fluor, Chlor oder Brom - steht,

$R^3$ für Wasserstoff Fluor, Chlor oder Brom steht,

$R^4$ für Wasserstoff Cyano, Fluor, Chlor, Brom oder für einen Rest der Formel $-O-R^6$, $-S-R^6$, $-C(O)-O-R^6$, $-C(O)-S-R^6$, $-NR^6R^7$ oder $-C(O)-NR^6R^7$ steht,

$R^5$ für Cyano oder Nitro steht und

X für Sauerstoff oder Schwefel steht, wobei

$R^6$ und $R^7$ unabhängig voneinander jeweils für Wasserstoff oder für gegebenenfalls einfach bis zweifach, gleich oder verschieden substituiertes, geradkettiges oder verzweigtes Alkyl mit 1 bis 6 Kohlenstoffatomen stehen, wobei als Substituenten infrage kommen:
Cyano, Carboxyl, Carbamoyl, jeweils geradkettiges oder verzweigtes Alkoxy, Alkoxyalkoxy, Halogenalkoxy (wobei Halogen für Fluor, Chlor und/oder Brom steht), Alkylthio, Alkylsulfinyl, Alkylsulfonyl, Alkylcarbonyl, Alkoxycarbonyl, Alkenyloxycarbonyl, Alkinyloxycarbonyl, Alkoxyalkoxycarbonyl, N-Alkylaminocarbonyl, N,N-Dialkylaminocarbonyl, Trialkylsilyl oder Alkylsulfonylaminocarbonyl mit jeweils bis zu 6 Kohlenstoffatomen in den einzelnen Alkyl- bzw. Alkenyl- oder Alkinylteilen oder Heterocyclyl, wobei als Heterocyclylrest ein fünf-oder sechsgliedriger, gesättigter oder ungesättigter Heterocyclus mit 1 bis 3 gleichen oder verschiedenen Heteroatomen - insbesondere

Stickstoff Sauerstoff und/oder Schwefel - steht;

$R^6$ und $R^7$ außerdem für geradkettiges oder verzweigtes Halogenalkyl mit 1 bis 6 Kohlenstoffatomen und 1 bis 13 gleichen oder verschiedenen Halogenatomen - insbesondere Fluor, Chlor und/oder Brom - stehen;

$R^6$ und $R^7$ außerdem für jeweils gegebenenfalls einfach bis fünffach, gleich oder verschieden durch Fluor, Chlor und/oder Brom substituiertes Alkenyl oder Alkinyl mit jeweils 2 bis 6 Kohlenstoffatomen stehen;

$R^6$ und $R^7$ außerdem für jeweils gegebenenfalls im Cycloalkylteil einfach bis vierfach, gleich oder verschieden durch Fluor, Chlor und/oder Brom und/oder jeweils geradkettiges oder verzweigtes Alkyl und/oder Alkoxy und/oder Alkoxycarbonyl mit jeweils 1 bis 3 Kohlenstoffatomen substituiertes Cycloalkyl, Cycloalkylalkyl oder Cycloalkyloxycarbonyl mit jeweils 3 bis 7 Kohlenstoffatomen im Cycloalkylteil und gegebenenfalls 1 bis 3 Kohlenstoffatomen im geradkettigen oder verzweigten Alkylteil stehen, oder

$R^6$ und $R^7$ für jeweils gegebenenfalls im Arylteil einfach bis fünffach, gleich oder verschieden substituiertes Arylalkyl oder Aryl mit jeweils 6 oder 10 Kohlenstoffatomen im Arylteil und gegebenenfalls 1 bis 3 Kohlenstoffatomen im geradkettigen oder verzweigten Alkylteil stehen, wobei als Arylsubstituenten jeweils infrage kommen:

Halogen, Cyano, Nitro, Hydroxy, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, geradkettiges oder verzweigtes Alkoxycarbonylalkyloxy mit 1 bis 4 Kohlenstoffatomen in den einzelnen Alkylteilen sowie gegebenenfalls einfach bis fünffach, gleich oder verschieden durch Halogen und/oder geradkettiges oder verzweigtes Alkyl oder Alkoxy mit jeweils 1 bis 4 Kohlenstoffatomen und/oder geradkettiges oder verzweigtes Halogenalkyl oder Halogenalkoxy mit jeweils 1 bis 4 Kohlenstoffatomen und 1 bis 9 gleichen oder verschiedenen Halogenatomen substituiertes Phenyl, wobei Halogen jeweils Fluor, Chlor und/oder Brom bedeutet.

**4.** Substituierte Triazolinone der allgemeinen Formel (I) gemäß Anspruch 1, dadurch gekennzeichnet, daß

$R^1$ für Wasserstoff oder für jeweils geradkettiges oder verzweigtes Alkyl, Alkoxy, Halogenalkyl oder Halogenalkoxy mit jeweils 1 bis 4 Kohlenstoffatomen und gegebenenfalls 1 bis 9 gleichen oder verschiedenen Halogenatomen - insbesondere Fluor, Chlor oder Brom - steht,

$R^2$ für Wasserstoff oder für jeweils geradkettiges oder verzweigtes Alkyl oder Halogenalkyl mit jeweils 1 bis 4 Kohlenstoffatomen und gegebenenfalls 1 bis 9 gleichen oder verschiedenen Halogenatomen - insbesondere Fluor, Chlor oder Brom - steht,

$R^3$ für Wasserstoff Fluor, Chlor oder Brom steht,

$R^4$ für Wasserstoff Cyano, Fluor, Chlor, Brom oder für einen Rest der Formel $-O-R^6$, $-S-R^6$, $-C(O)-O-R^6$, $-C(O)-S-R^6$, $-NR^6R^7$ oder $-C(O)-NR^6R^7$ steht,

$R^5$ für Cyano oder Nitro steht und

X für Sauerstoff oder Schwefel steht, wobei

$R^6$ und $R^7$ unabhängig voneinander jeweils für Wasserstoff oder für gegebenenfalls einfach substituiertes, geradkettiges oder verzweigtes Alkyl mit 1 bis 4 Kohlenstoffatomen stehen, wobei als Substituenten infrage kommen:

Cyano, Carboxyl, Carbamoyl, jeweils geradkettiges oder verzweigtes Alkoxy, Alkoxyalkoxy, Halogenalkoxy (wobei Halogen für Fluor, Chlor und/oder Brom steht), Alkylthio, Alkylsulfinyl, Alkylsulfonyl, Alkylcarbonyl, Alkoxycarbonyl, Alkenyloxycarbonyl, Alkinyloxycarbonyl, Alkoxyalkoxycarbonyl, N-Alkylaminocarbonyl, N,N-Dialkylaminocarbonyl, Trialkylsilyl oder Alkylsulfonylaminocarbonyl mit jeweils bis zu 4 Kohlenstoffatomen in den einzelnen Alkyl- bzw. Alkenyl- oder Alkinylteilen, oder Heterocyclyl, wobei als Heterocyclylrest ein fünf-oder sechsgliedriger, gesättigter oder ungesättigter Heterocyclus mit 1 bis 3 gleichen oder verschiedenen Heteroatomen - insbesondere Stickstoff, Sauerstoff und/oder Schwefel - steht;

$R^6$ und $R^7$ außerdem für geradkettiges oder verzweigtes Halogenalkyl mit 1 bis 4 Kohlenstoffatomen und 1 bis 9 gleichen oder verschiedenen Halogenatomen - insbesondere Fluor, Chlor und/oder Brom - stehen;

| | |
|---|---|
| $R^6$ und $R^7$ | außerdem für jeweils gegebenenfalls einfach bis dreifach, gleich oder verschieden durch Halogen - insbesondere Fluor, Chlor und/oder Brom - substituiertes Alkenyl oder Alkinyl mit jeweils 3 bis 5 Kohlenstoffatomen stehen; |
| $R^6$ und $R^7$ | außerdem für jeweils gegebenenfalls im Cycloalkylteil einfach oder zweifach, gleich oder verschieden durch Fluor, Chlor, Brom, Methyl, Ethyl, Methoxy, Ethoxy, Methoxycarbonyl und/oder Ethoxycarbonyl substituiertes Cycloalkyl, Cycloalkylalkyl oder Cycloalkyloxycarbonyl mit jeweils 3 bis 6 Kohlenstoffatomen im Cycloalkylteil und gegebenenfalls 1 oder 2 Kohlenstoffatomen im Alkylteil stehen, oder |
| $R^6$ und $R^7$ | für jeweils gegebenenfalls im Phenylteil einfach bis dreifach, gleich oder verschieden substituiertes Phenylalkyl oder Phenyl mit gegebenenfalls 1 oder 2 Kohlenstoffatomen im Alkylteil stehen, wobei als Phenylsubstituenten jeweils infrage kommen: Halogen, Cyano, Nitro, Hydroxy, jeweils geradkettiges oder verzweigtes Alkyl, Alkoxy, Alkylthio, Alkylsulfinyl oder Alkylsulfonyl mit jeweils 1 bis 3 Kohlenstoffatomen oder jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen sowie geradkettiges oder verzweigtes Alkoxycarbonylalkyloxy mit 1 bis 2 Kohlenstoffatomen in den einzelnen Alkylteilen, wobei Halogen jeweils Fluor, Chlor und/oder Brom bedeutet. |

**5.** Verfahren zur Herstellung substituierter Triazolinone der allgemeinen Formel (I)

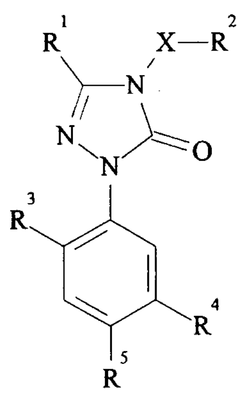

(I)

in welcher

| | |
|---|---|
| $R^1$ | für Wasserstoff, Alkyl, Alkoxy, Halogenalkyl oder Halogenalkoxy steht, |
| $R^2$ | für Wasserstoff, Alkyl oder Halogenalkyl steht, |
| $R^3$ | für Wasserstoff oder Halogen steht, |
| $R^4$ | für Wasserstoff, Cyano, Halogen oder für einen Rest der Formel - O-$R^6$, -S-$R^6$, -C(O)-O-$R^6$, -C(O)-S-$R^6$, -N$R^6R^7$ oder -C(O)-N$R^6R^7$ steht, |
| $R^5$ | für Cyano oder Nitro steht und |
| X | für Sauerstoff oder Schwefel steht, wobei |
| $R^6$ und $R^7$ | unabhängig voneinander jeweils für Wasserstoff oder für jeweils gegebenenfalls substituiertes Alkyl, Alkenyl, Alkinyl, Cycloalkyl, Cycloalkylalkyl, Cycloalkoxycarbonyl, Aryl oder Arylalkyl stehen, |

dadurch gekennzeichnet, daß man

a) 1H-Triazolinone der Formel (II),

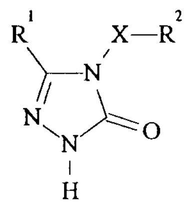

(II)

in welcher

$R^1$, $R^2$ und X die oben angegebenen Bedeutungen haben,

mit Halogenbenzol-Derivaten der Formel (III),

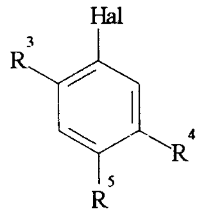

(III)

in welcher

$R^3$, $R^4$ und $R^5$ die oben angegebenen Bedeutungen haben und

Hal für Halogen steht,

gegebenenfalls in Gegenwart eines Verdünnungsmittels und gegebebenfalls in Gegenwart eines Reaktionshilfsmittels umsetzt, oder daß man

b) substituierte Triazolinone der Formel (Ia),

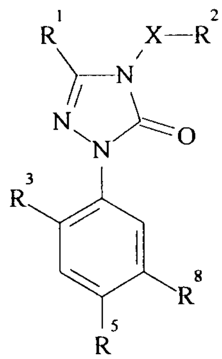

(Ia)

in welcher

$R^1$, $R^2$, $R^3$, $R^5$ und X die oben angegebenen Bedeutungen haben und

$R^8$ für Halogen steht,

mit Nukleophilen der Formel (IV),

$R^9$-Z-H (IV)

in welcher

Z für Sauerstoff, Schwefel oder für den Rest -N($R^7$)- steht,

$R^9$ für jeweils gegebenenfalls substituiertes Alkyl, Alkenyl, Alkinyl, Cycloalkyl oder Aryl steht und

$R^7$ die oben angegebene Bedeutung hat,

gegebenenfalls in Gegenwart eines Verdünnungsmittels und gegebenenfalls in Gegenwart eines Reaktionshilfsmittels umsetzt, oder daß man

c) substituierte Triazolinone der Formel (V),

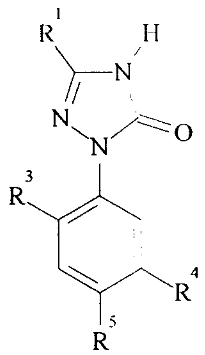

(V)

in welcher
$R^1$, $R^3$, $R^4$ und $R^5$ die oben angegebenen Bedeutungen haben,
mit Sulfenylierungsmitteln der Formel (VI),

$R^2$-S-Hal (VI)

in welcher
$R^2$ die oben angegebene Bedeutung hat und
Hal für Halogen steht,
gegebenenfalls in Gegenwart eines Verdünnungsmittels und gegebenenfalls in Gegenwart eines Reaktionshilfsmittels umsetzt.

6. Verfahren zur Herstellung von herbiziden Mitteln, dadurch gekennzeichnet, daß man substituierte Triazolinone der allgemeinen Formel (I) gemäß der Ansprüche 1 bis 5 mit Streckmitteln und/oder oberflächenaktiven Mitteln vermischt.

7. Verfahren zur Bekämpfung von unerwünschten Pflanzen, dadurch gekennzeichnet, daß man substituierte Triazolinone der allgemeinen Formel (I) gemäß der Ansprüche 1 bis 5 auf Pflanzen und/oder ihren Lebensraum einwirken läßt.

8. Verwendung von substituierten Triazolinonen der allgemeinen Formel (I) gemäß der Ansprüche 1 bis 5 zur Bekämpfung von unerwünschten Pflanzen.

9. Herbizide Mittel, gekennzeichnet durch einen Gehalt an mindestens einem substituierten Triazolinon der allgemeinen Formel (I) gemäß der Ansprüche 1 bis 5.

Europäisches Patentamt

# EUROPÄISCHER RECHERCHENBERICHT

Nummer der Anmeldung

EP 93 11 8206

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 370 332 (BAYER AG) 30. Mai 1990 * Ansprüche 1-7; Beispiele 5,6,25,26 * | 1-9 | C07D249/14 A01N43/653 |
| Y | US-A-5 041 155 (FMC CORPORATION) 20. August 1991 * Spalte 1, Zeile 28 - Spalte 2, Zeile 41; Ansprüche 1-5 * | 1-9 | |
| A | CHEMICAL ABSTRACTS, vol. 108, no. 9, 29. Februar 1988, Columbus, Ohio, US; abstract no. 75311j, W. SCHAPER 'Cyclic hydroxamic acids: synthesis of 4-hydroxy-1H-1,2,4-triazolin-5-ones' Seite 673 ; * Zusammenfassung * & Arch. Pharm. (Weinheim, Ger.) 320 (11) 1167-73 (1987) | 1-9 | |
| A | CHEMICAL ABSTRACTS, vol. 72, no. 19, 11. Mai 1970, Columbus, Ohio, US; abstract no. 100656y, G. MATOLCSY, B. BORDAS 'Synthesis and antifungal activity of some new N-trichloromethylthio derivatives' Seite 372 ; * Zusammenfassung * & Acta Phytopathol. 4 (2-3) 197-201 (1969) | 1-9 | RECHERCHIERTE SACHGEBIETE (Int. Cl.5) C07D A01N |

Der vorliegende Recherchenbericht wurde für alle Patentansprüche erstellt

| Recherchenort | Abschlußdatum der Recherche | Prüfer |
|---|---|---|
| MUENCHEN | 11 JANUAR 1994 | HERZ C.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)